# EUROPEAN PATENT SPECIFICATION

(11) **EP 4 589 299 B1**
(45) Date of publication and mention of the grant of the patent: **27.05.2026**
(21) Application number: 25173406.7
(22) Date of filing: 05.12.2022
(51) Int. Cl.: G01N 33/80, G01N 33/543

(54) **APPLICATION OF BLOOD GROUP ANTIGEN TRISACCHARIDE CONJUGATE IN THE DETECTION OF BLOOD GROUP ANTIBODIES**
VERWENDUNG EINES BLUTGRUPPENANTIGEN-TRISACCHARID-KONJUGATS BEIM NACHWEIS VON BLUTGRUPPENANTIKÖRPERN
APPLICATION D'UN CONJUGUÉ ANTIGÈNE DE GROUPE SANGUIN TRISACCHARIDE DANS LA DÉTECTION D'ANTICORPS DE GROUPE SANGUIN

(30) Priority: 18.10.2022 CN 202211269823
(43) Date of publication of application: 23.07.2025
(62) Divisional of application: 22940937.0
(73) Proprietor: Tianjin Dexiang Biotechnology Co., Ltd., Tianjin 300462 (CN)
(72) Inventor: PANG, Wei, Tianjin, 300462 (CN); HUANG, Zhi Gang, Tianjin, 300462 (CN); WEI, Li Qiao, Tianjin, 300462 (CN)
(74) Representative: Novagraaf Group

(56) References cited:
- CN-A- 108 659 115
- JP-A- 2000 088 847
- US-A1- 2006 057 562

## Description

### Technical Field

. The present invention belongs to the field of blood group antibody detection, and relates to use of a blood group antigenic trisaccharide conjugate in the blood group antibody detection, in particular to use of a blood group antigenic trisaccharide A analogue-protein conjugate and a blood group antigenic trisaccharide B analogue-protein conjugate in the blood group antibody detection.

### Background

. Blood grouping is a primary job before clinical blood transfusion. When blood is transfused into the blood of different blood groups, due to the agglutination of antigens and antibodies, hemolysis will occur, which may endanger human life safety. Thus, correct blood grouping is a prerequisite for ensuring the safety of blood transfusion. Current blood grouping methods include forward grouping and reverse grouping. Erythrocyte antigens are detected in the forward grouping, and antibodies in the serum are detected in the reverse grouping, wherein the detection of human A, B, O blood group antigens/antibodies is the most important. Human blood can be divided into different blood groups according to different surface antigens of erythrocyte membranes. Group A blood has a blood group antigen A (hereinafter referred to as "antigen A") on the erythrocyte with a blood group antibody B (hereinafter referred to as "antibody B") in the serum. Group B blood has a blood group antigen B (hereinafter referred to as "antigen B") on the erythrocyte with a blood group antibody A (hereinafter referred to as "antibody A") in the serum. Group AB blood has both the antigen A and the antigen B on the erythrocyte without the antibody A or the antibody B in the serum. Group O blood has neither the antigen A nor the antigen B on the erythrocyte with both the antibody A and the antibody B in the serum.

. At present, an agglutination method is generally used in common forward grouping and reverse grouping of blood groups, and its principle is to use blood group antigens to react with antibodies to cause erythrocyte agglutination that is visible with naked eyes, to judge results. During forward grouping, an IgM anti-A or anti-B reagent is used to detect erythrocyte antigens of samples. During reverse grouping, a known group A or B erythrocyte reagent is used to determine IgM blood group antibodies in the serum sample. However, since fresh erythrocytes are not easy to preserve (the antigenicity of blood group antigens on surfaces of the erythrocytes gradually decreases with the extension of preservation time), the group A or B erythrocyte reagent for reverse grouping detection has higher requirements for preservation conditions and transportation conditions, which increases the cost for the reverse grouping detection and limits the use of the reverse grouping detection. Some technical solutions in the prior art attempt to extract natural blood group antigens (for example, to extract natural antigens A and antigens B from the erythrocyte membranes) to replace intact erythrocytes in the reverse grouping detection. However, the relevant extraction and preparation processes are often more complex and uneasy for mass production, and the extracted natural blood group antigens still have higher requirements for the preservation conditions. Artificial synthesis of blood group antigens is one of the research directions to solve the above technical problems, but it is currently known that the artificially synthesized blood group antigens have technical problems in specificity, affinity, stability, etc. and cannot be effectively applied to the reverse grouping detection.

US2006057562A1 relates to a method for detecting a Norwalk-Like Virus (NLV) in a biological sample, comprising the steps of: obtaining a biological sample suspected of containing a NLV; contacting the biological sample with at least one human histo-blood group antigen to allow formation of a complex of the NLV with the antigen; and detecting the antigen NLV complex.

JP2000088847A relates to analyzing a blood type using an absorptive carrier with a synthetic blood type antigen having an antigen property chemically synthesized and includes an ABO blood type antigen and an Rh blood type antigen.

CN108659115A relates to a synthetic method for a blood group antigen trisaccharide A analogue BSA derivative, the method comprising: (1) performing a cycloaddition reaction on a Cu catalysis blood group antigen trisaccharide A analogue and an azide with a terminal position connected with carboxyl to form an intermediate product 1,4-disubstituted-1,2-triazole compound; and (2) performing a coupling reaction on the cycloaddition product obtained in the step (1) and BSA to synthesize an immunoconjugate.

### Summary

. The invention is as defined in the claims. The present disclosure provides use of a blood group antigenic trisaccharide conjugate in preparation of a blood group antibody detection reagent, the blood group antigenic trisaccharide conjugate is a blood group antigenic trisaccharide analogue-protein conjugate; the blood group antigenic trisaccharide analogue includes a blood group antigenic trisaccharide B analogue; the blood group antigenic trisaccharide B analogue is conjugated to a keyhole limpet hemocyanin (KLH) or a bovine serum albumin (BSA) to form a blood group antigenic trisaccharide B analogue-protein conjugate; when the blood group antigenic trisaccharide B analogue is a type B2, a conjugation ratio of the blood group antigenic trisaccharide B analogue to the keyhole limpet hemocyanin is 5:1 - 40:1, and a conjugation ratio of the blood group antigenic trisaccharide B analogue to the bovine serum albumin is 20:1; when the blood group antigenic trisaccharide B analogue is a type B1, a type B3 or a type B4, a conjugation ratio of the blood group antigenic trisaccharide B analogue to the keyhole limpet hemocyanin is 40:1.

. In the disclosure, when the blood group antigenic trisaccharide B analogue is the type B2, a conjugation ratio of the blood group antigenic trisaccharide B analogue to the keyhole limpet hemocyanin is 20:1.

. In the disclosure, the blood group antigenic trisaccharide analogue further includes a blood group antigenic trisaccharide A analogue; the blood group antigenic trisaccharide A analogue is conjugated to the keyhole limpet hemocyanin or the bovine serum albumin to form a blood group antigenic trisaccharide A analogue-protein conjugate.

### . Beneficial technical effects

. 1) Experiments have proved that the above blood group antigenic trisaccharide A analogue (A1-A4) protein-conjugate (conjugated to a BSA or a KLH) and the above blood group antigenic trisaccharide B analogue (B1-B4) protein-conjugate (conjugated to a BSA or a KLH) (see Tables 7a and 7b) used in the technical solutions of the present invention have good specificity, can distinguish and identify the corresponding blood group antibodies with high detection accuracy as the artificially synthesized blood group antigens, and can be applied to the detection of various types of blood group antibodies, which have great potential clinical application value. Especially in the immunochromatographic detection, a part of the blood group antigenic trisaccharide analogue-protein conjugates used in the technical solutions of the present invention can be stably immobilized on test strip, and efficiently capture the corresponding blood group antibodies; the color development effect (for example, color development by colloidal gold labeling) is easily identifiable and visible with naked eyes, thereby having the potential to be applied to scenarios such as rapid blood group screening, point-of-care testing (POCT) and blood group self-examination for ordinary people.
. 2) The blood group antigenic trisaccharide analogue-protein conjugates used in the present invention are synthesized by a chemical synthesis process, which is relatively easy to achieve industrial-grade mass production, and can be used as a blood group antigenic raw material in various experimental systems to replace the erythrocyte reagent or natural blood group antigens in, for example, the reverse grouping detection, thereby avoiding the cumbersome processes for extracting and preparing the natural blood group antigens and reducing the cost for blood group detection.

### Description of Drawings

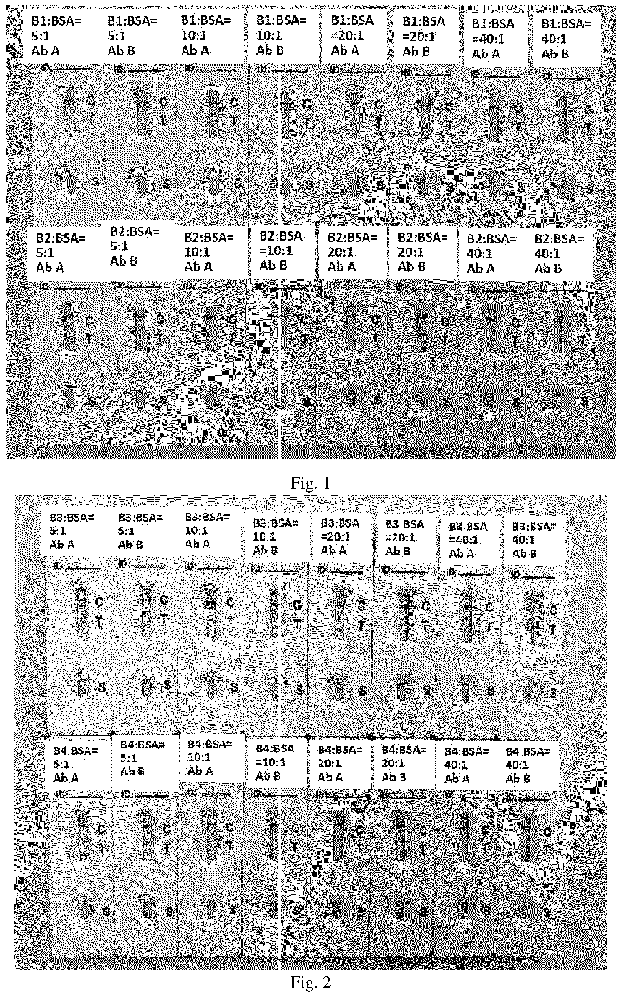

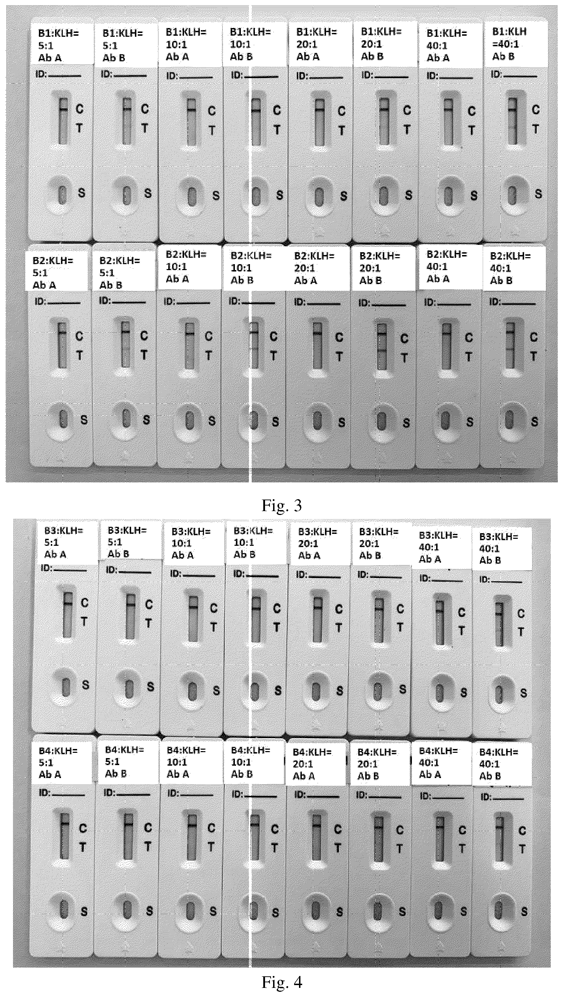

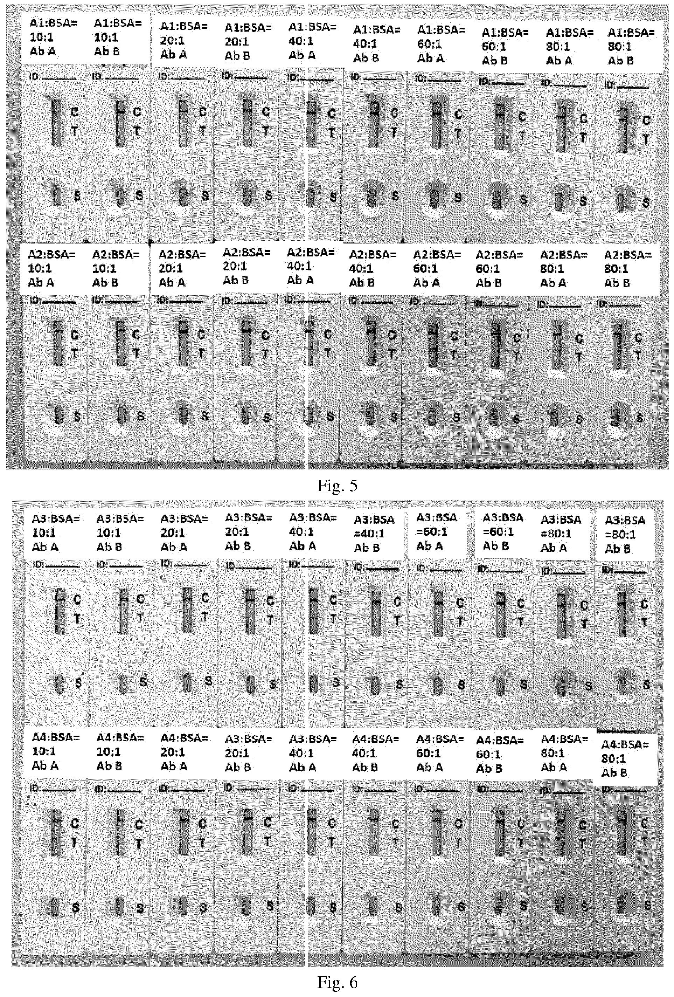

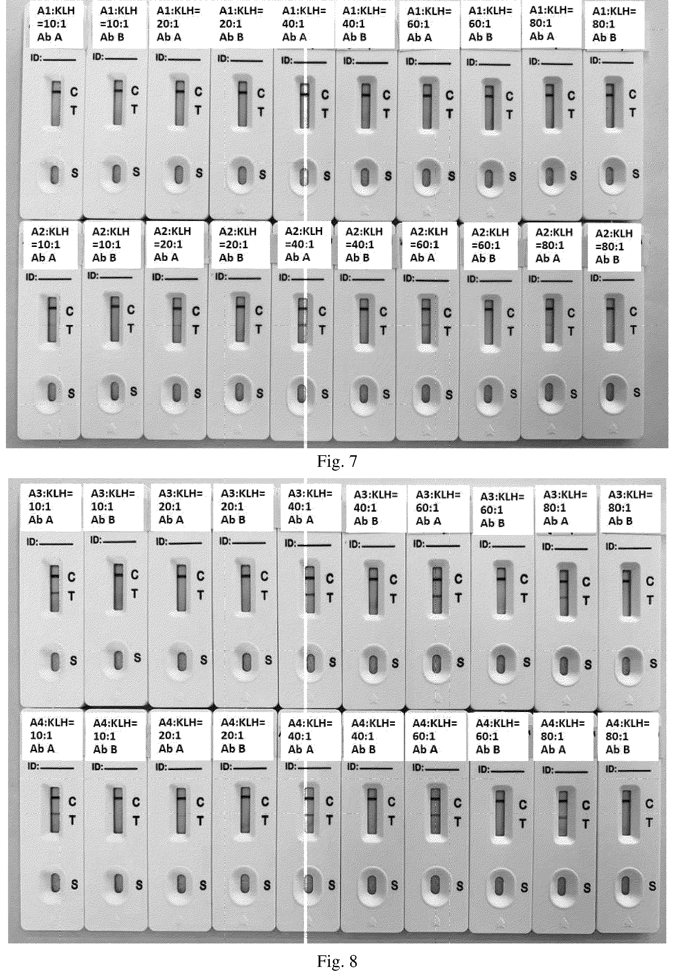

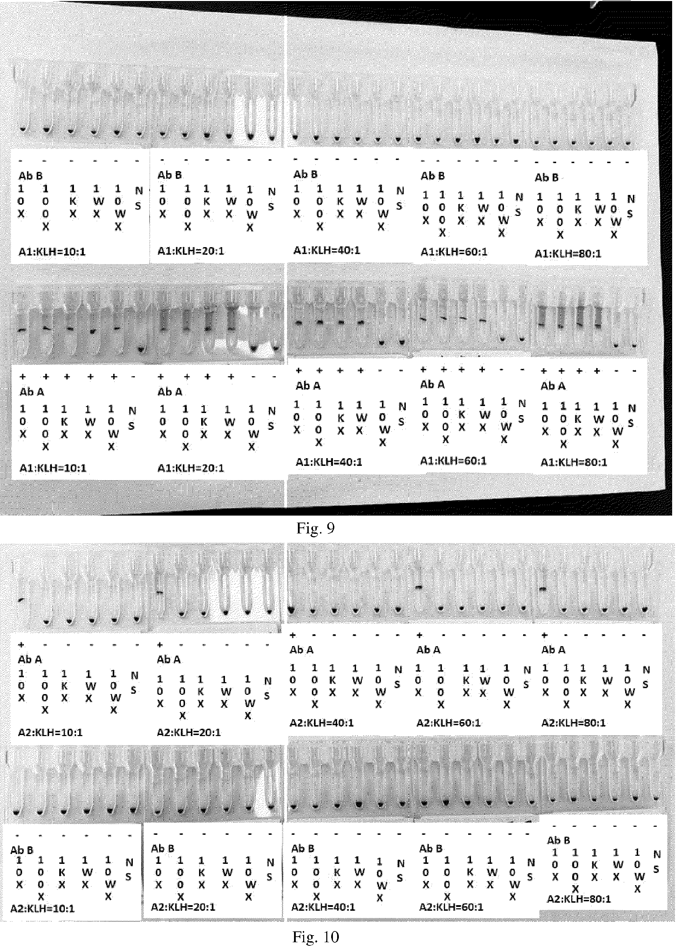

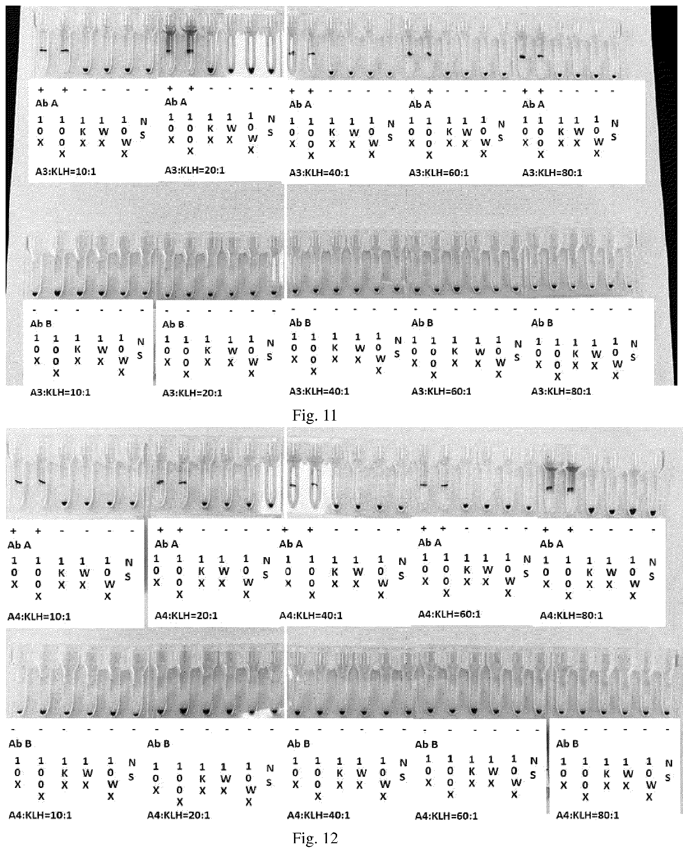

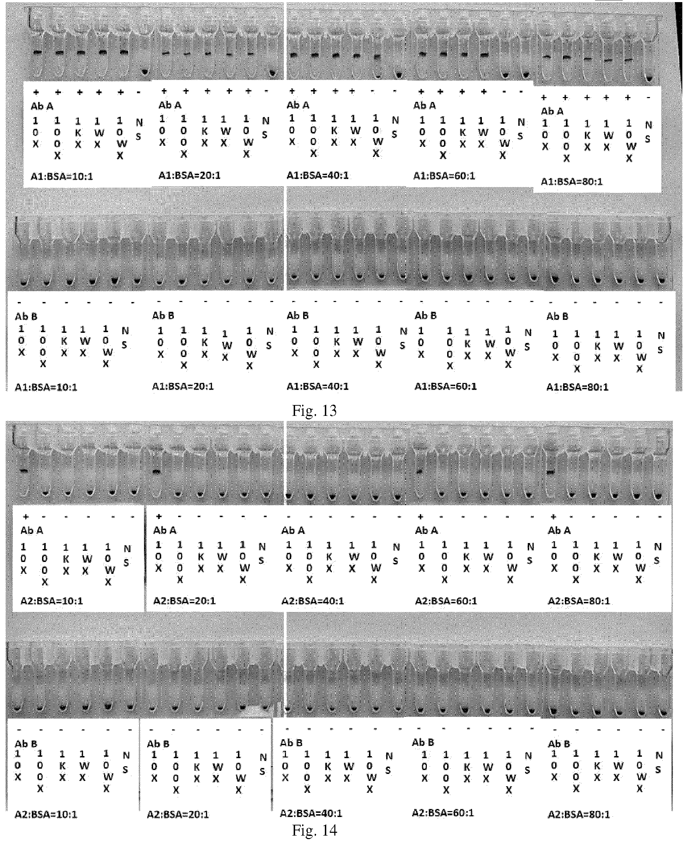

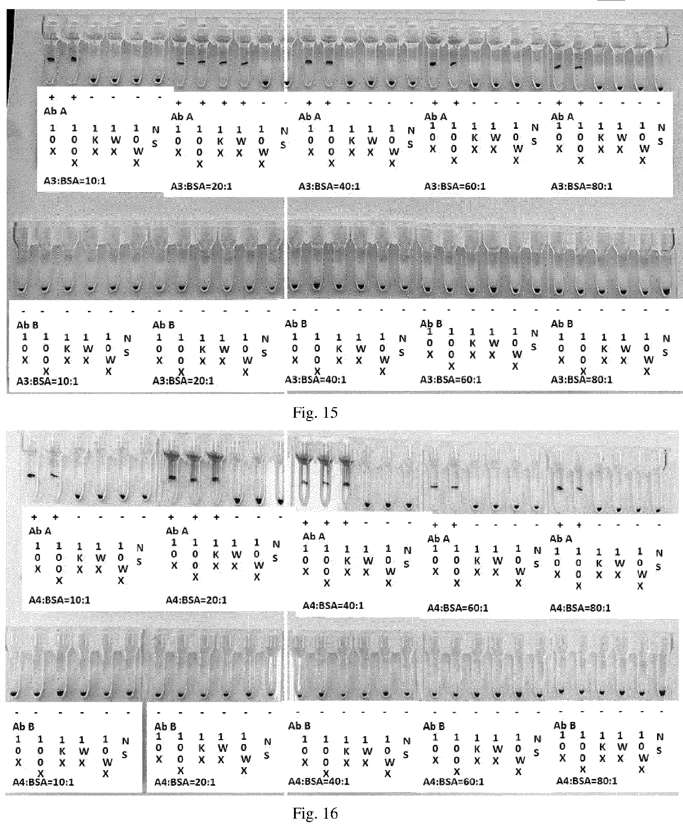

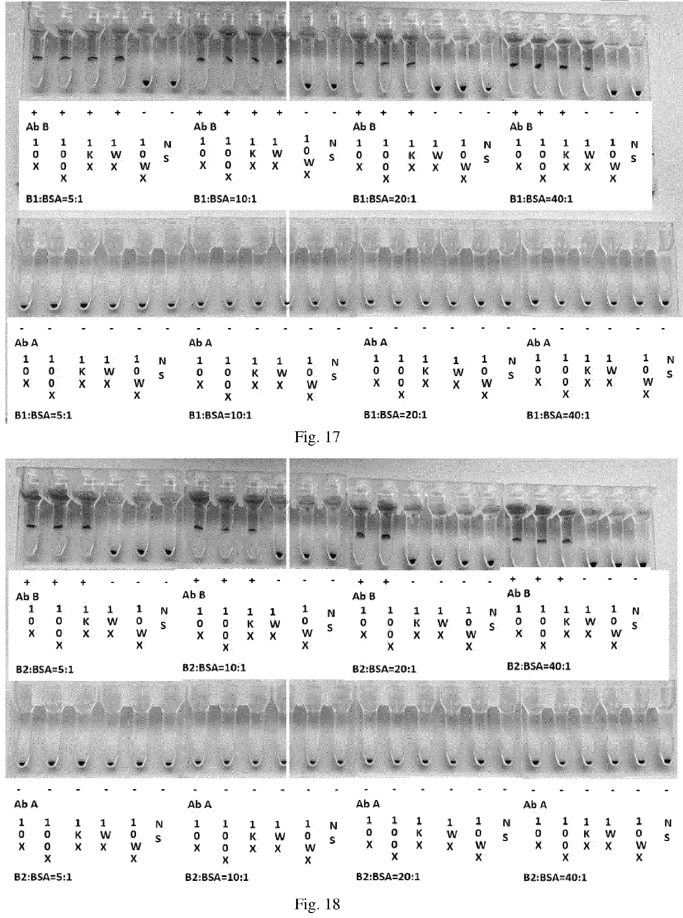

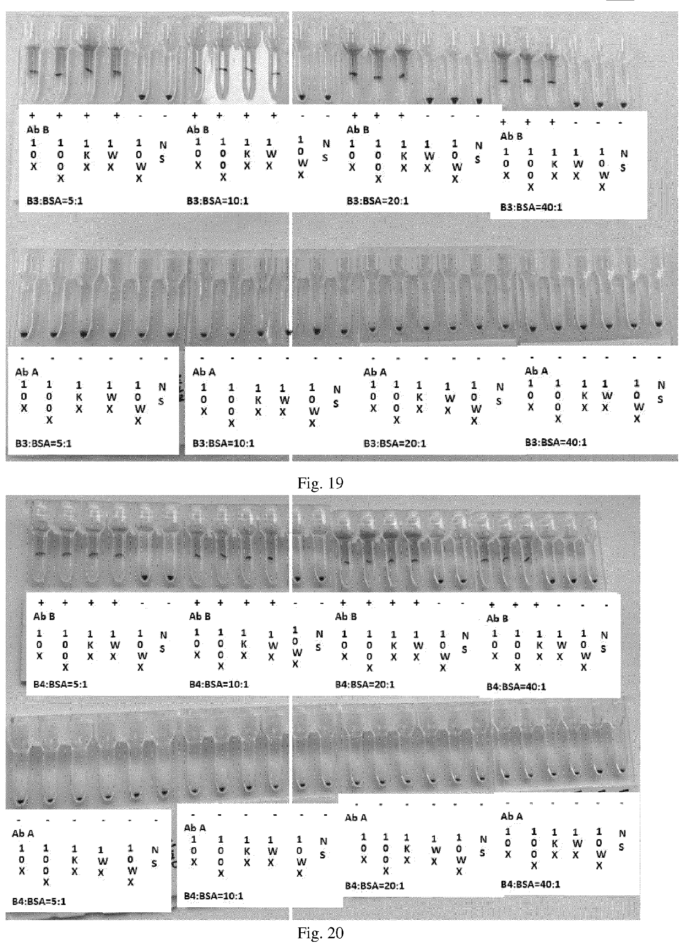

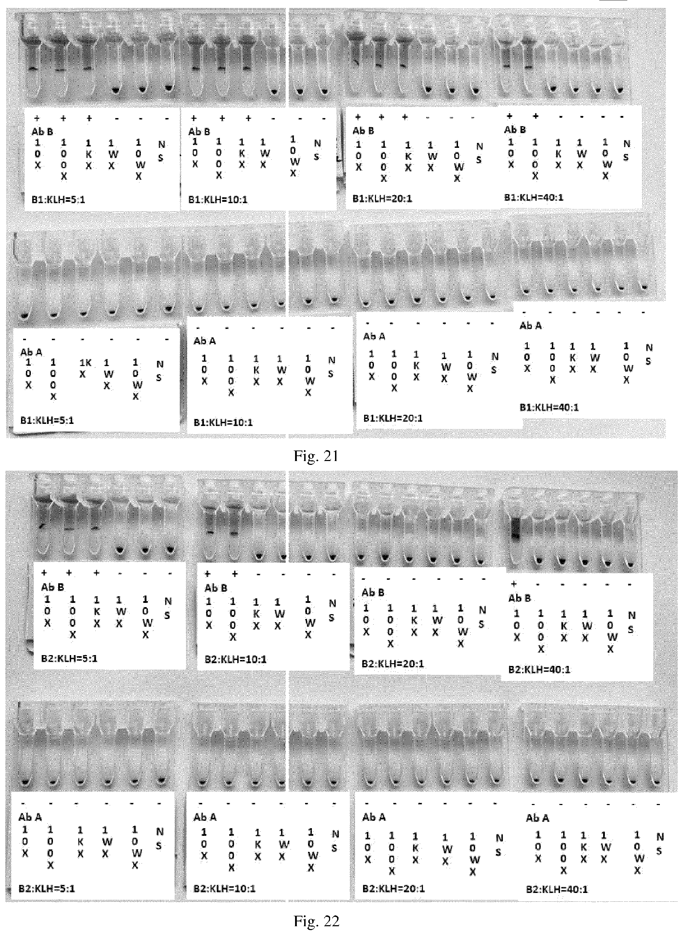

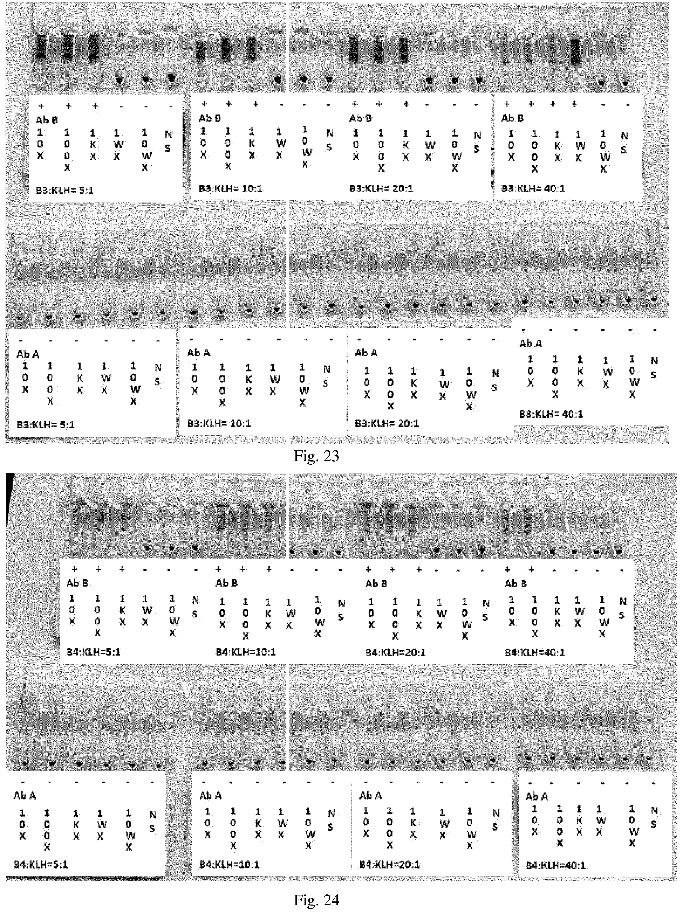

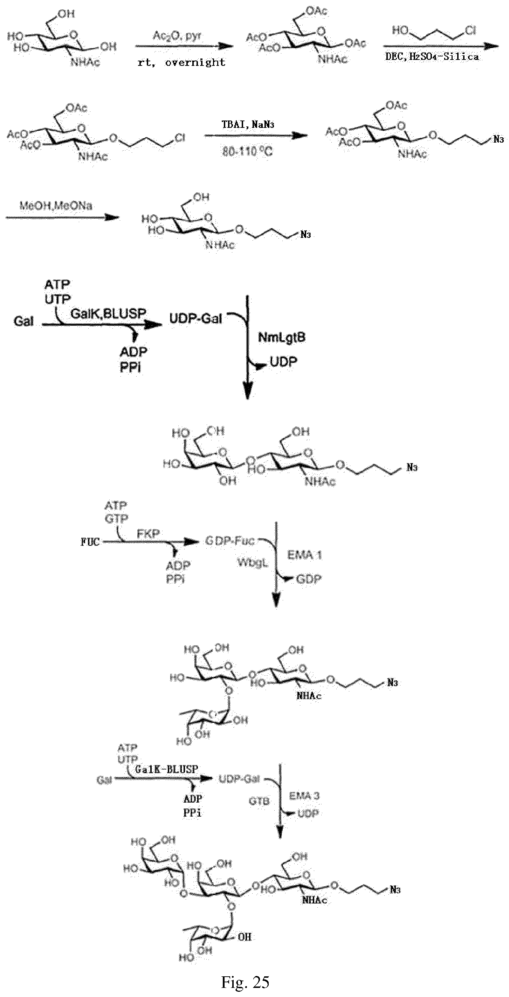

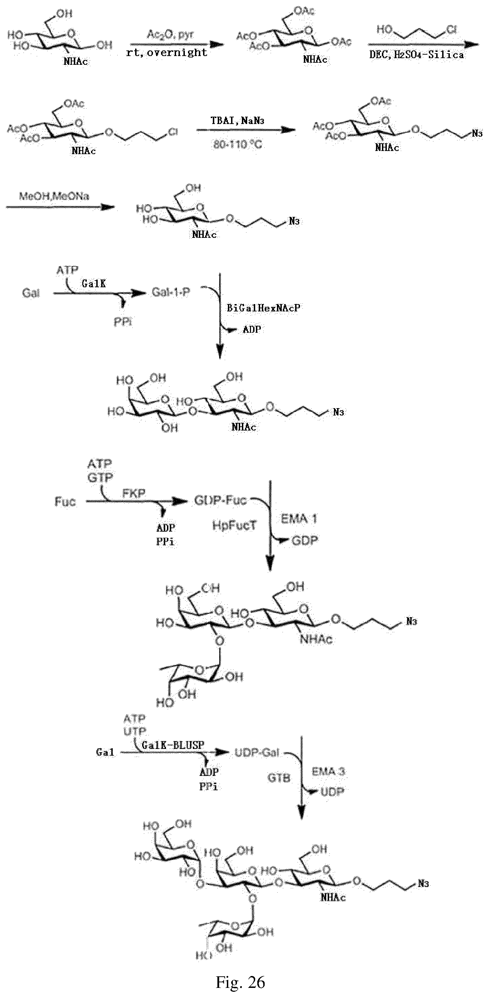

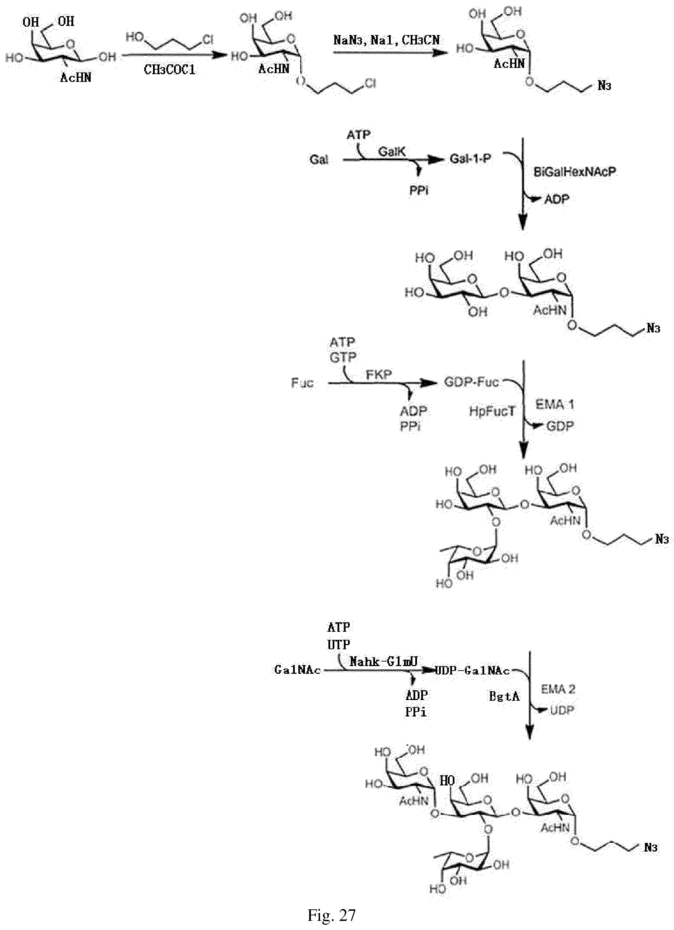

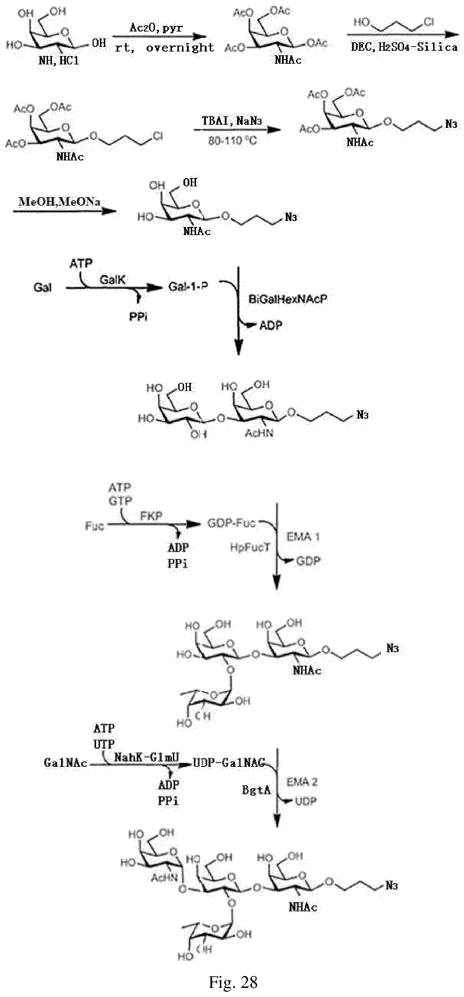

. To make the embodiments of the present invention or the technical solutions in the prior art clearer, the drawings required to be used in the description of the embodiments or the prior art will be briefly introduced below. It is obvious that the drawings described below are some embodiments of the present invention, and that other drawings can be obtained from these drawings for those of ordinary skill in the art without making inventive effort.
. Fig. 1 is a diagram showing a color development effect of a conjugate of B1-B2 to a BSA on a nitrocellulose membrane (NC membrane) in an embodiment of the present invention;
. Fig. 2 is a diagram showing a color development effect of a conjugate of B3-B4 to the BSA on the NC membrane in an embodiment of the present invention;
. Fig. 3 is a diagram showing a color development effect of a conjugate of B1-B2 to a KLH on the NC membrane in an embodiment of the present invention;
. Fig. 4 is a diagram showing a color development effect of a conjugate of B3-B4 to the KLH on the NC membrane in an embodiment of the present invention;
. Fig. 5 is a diagram showing a color development effect of a conjugate of A1-A2 to the BSA on the NC membrane in an embodiment of the present invention;
. Fig. 6 is a diagram showing a color development effect of a conjugate of A3-A4 to the BSA on the NC membrane in an embodiment of the present invention;
. Fig. 7 is a diagram showing a color development effect of a conjugate of A1-A2 to the KLH on the NC membrane in an embodiment of the present invention;
. Fig. 8 is a diagram showing a color development effect of a conjugate of A3-A4 to the KLH on the NC membrane in an embodiment of the present invention;
. Fig. 9 is a diagram showing effect of a reverse blood grouping detection card for detection of antibodies A and B with A1-KLH;
. Fig. 10 is a diagram showing effect of a reverse blood grouping detection card for detection of antibodies A and B with A2-KLH;
. Fig. 11 is a diagram showing effect of a reverse blood grouping detection card for detection of antibodies A and B with A3-KLH;
. Fig. 12 is a diagram showing effect of a reverse blood grouping detection card for detection of antibodies A and B with A4-KLH;
. Fig. 13 is a diagram showing effect of a reverse blood grouping detection card for detection of antibodies A and B with A1-BSA;
. Fig. 14 is a diagram showing effect of a reverse blood grouping detection card for detection of antibodies A and B with A2-BSA;
. Fig. 15 is a diagram showing effect of a reverse blood grouping detection card for detection of antibodies A and B with A3-BSA;
. Fig. 16 is a diagram showing effect of a reverse blood grouping detection card for detection of antibodies A and B with A4-BSA;
. Fig. 17 is a diagram showing effect of a reverse blood grouping detection card for detection of antibodies A and B with B1-BSA;
. Fig. 18 is a diagram showing effect of a reverse blood grouping detection card for detection of antibodies A and B with B2-BSA;
. Fig. 19 is a diagram showing effect of a reverse blood grouping detection card for detection of antibodies A and B with B3-BSA;
. Fig. 20 is a diagram showing effect of a reverse blood grouping detection card for detection of antibodies A and B with B4-BSA;
. Fig. 21 is a diagram showing effect of a reverse blood grouping detection card for detection of antibodies A and B with B1-KLH;
. Fig. 22 is a diagram showing effect of a reverse blood grouping detection card for detection of antibodies A and B with B2-KLH;
. Fig. 23 is a diagram showing effect of a reverse blood grouping detection card for detection of antibodies A and B with B3-KLH;
. Fig. 24 is a diagram showing effect of a reverse blood grouping detection card for detection of antibodies A and B with B4-KLH;
. Fig. 25 is a diagram showing a synthesis route of a blood group antigenic trisaccharide B analogue type I according to the present invention;
. Fig. 26 is a diagram showing a synthesis route of a blood group antigenic trisaccharide B analogue type II according to the present invention;
. Fig. 27 is a diagram showing a synthesis route of a blood group antigenic trisaccharide B analogue type III according to the present invention; and
. Fig. 28 is a diagram showing a synthesis route of a blood group antigenic trisaccharide B analogue type IV according to the present invention.

### Detailed Description

. To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

. As used herein, the term "about", typically means +/-5% of the stated value, more typically +/-4% of the stated value, more typically +/-3% of the stated value, more typically, +/-2% of the stated value, even more typically +/-1% of the stated value, and even more typically +/-0.5% of the stated value.

. Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

. The "blood group antigenic trisaccharide A analogue" according to the present invention refers to four compounds with basic chemical formulas as shown in chemical formulas A1-A4 and derivatives thereof, which are a type I antigenic A compound (chemical formula A1, hereinafter referred to as "A1" or "a type A1"), a type II antigenic A compound (chemical formula A2, hereinafter referred to as "A2" or "a type A2"), a type III antigenic A compound (chemical formula A3, hereinafter referred to as "A3" or "a type A3") and a type IV antigenic A compound (chemical formula A4, hereinafter referred to as "A4" or "a type A4"), respectively.

.

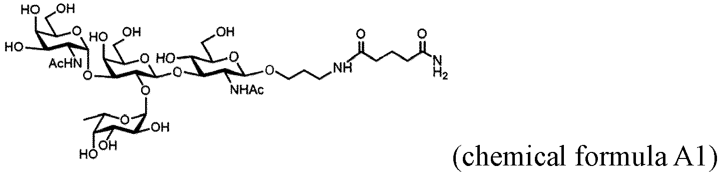

.

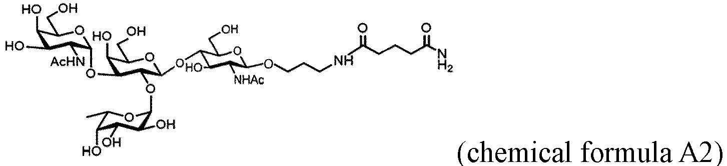

.

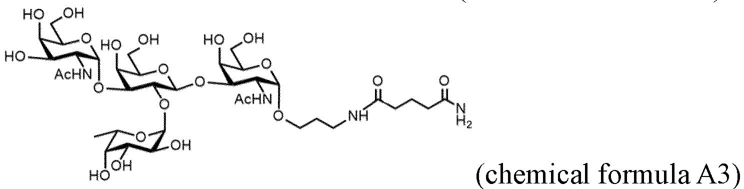

.

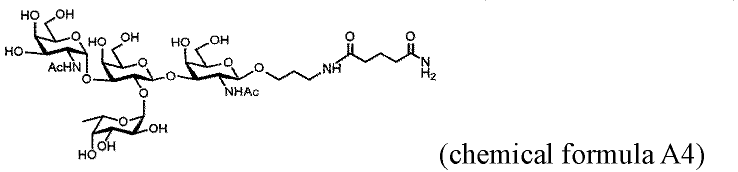

. The "blood group antigenic trisaccharide B analogue" according to the present invention refers to four compounds with basic chemical formulas as shown in chemical formulas B1-B4 and derivatives thereof, which are a type I antigenic B compound (chemical formula B1, hereinafter referred to as "B1" or "a type B1"), a type II antigenic B compound (chemical formula B2, hereinafter referred to as "B2" or "a type B2"), a type III antigenic B compound (chemical formula B3, hereinafter referred to as "B3" or "a type B3") and a type IV antigenic B compound (chemical formula B4, hereinafter referred to as "B4" or "a type B4"), respectively.

.

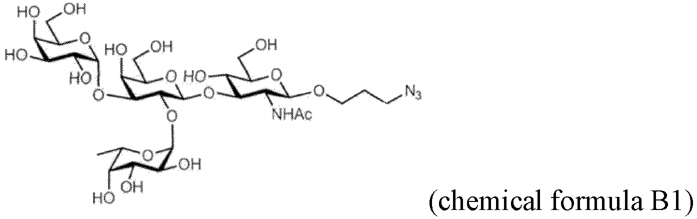

.

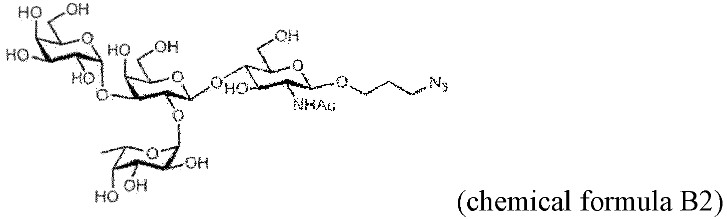

.

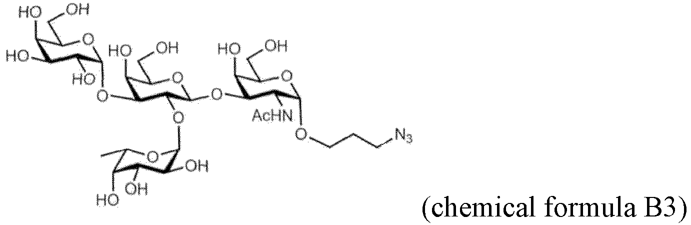

.

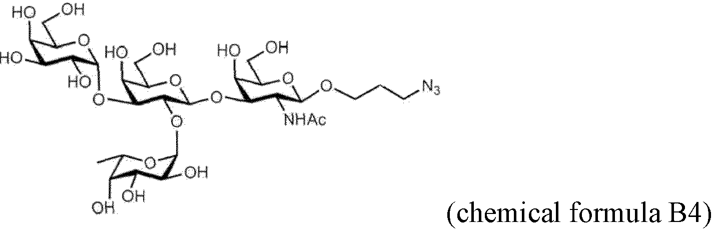

. The "blood group antigenic trisaccharide analogue-protein conjugate" according to the present invention refers to a protein conjugate formed by conjugation of the above blood group antigenic trisaccharide A analogue to a BSA or a KLH (hereinafter referred to as "A-BSA" and "A-KLH"), and a protein conjugate formed by conjugation of the above blood group antigenic trisaccharide B analogue to a BSA or a KLH (hereinafter referred to as "B-BSA" and "B-KLH").

### Embodiment 1 Preparation of the blood group antigenic trisaccharide analogue-protein conjugate

### 1.1 Preparation of the blood group antigenic trisaccharide A analogue-protein conjugate

. In some embodiments, according to a modular assembly strategy method from the Bovin research group (Drouillar S, et al., Large-scale synthesis of H-antigen oligosaccharides by expressing helicobacter pylorial,2-fucosyltransferase in metabolically engineered Escherichia coli cells[J]. Angew Chem, 2006, 118(11):1810-1812), two target sugar building blocks are synthesized first, and then a target sugar chain is synthesized by glycosylation. Main reaction steps are shown as follows:

.

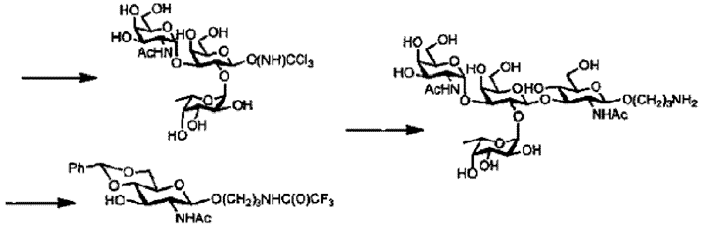

. Then terminal site of the target sugar chain generated by the above method undergo an addition reaction with butyrylamide connected with a carboxyl group to generate the antigenic trisaccharide A analogue of the present invention, four types (A1-A4) in total, with chemical structure formulas as shown in chemical formulas A1-A4. Finally, a linear linker arm with low immunogenicity, good reactivity and a high conjugation ratio is used to conjugate the blood group antigenic trisaccharide A analogue to an amino group on a lysine residue of the BSA or the KLH (for example, a section of alkane chain connected with an azide group is pre-loaded on a starting substrate during synthesis of the sugar chain, as the linker arm, and then a reagent that make both ends of the linker arm form activated esters is used to achieve the conjugation of the blood group antigenic trisaccharide analogue to a carrier protein), thereby synthesizing the blood group antigenic trisaccharide A analogue-protein conjugates (A1-BSA, A2-BSA, A3-BSA, A4-BSA; A1-KLH, A2-KLH, A3-KLH, A4-KLH) of the present invention with chemical formulas thereof shown as follows:
. A1-BSA
   . 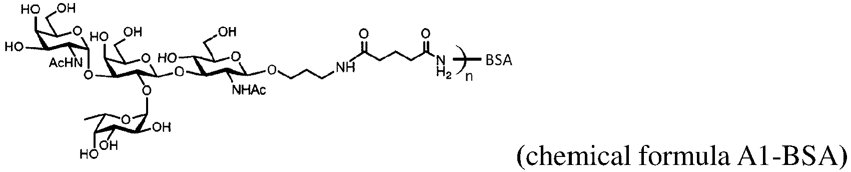
. A2-BSA
   . 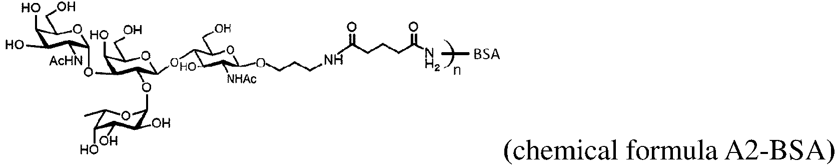
. A3-BSA
   . 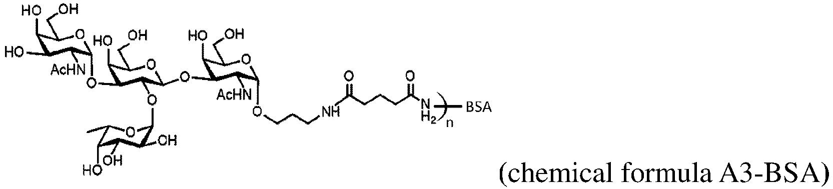
. A4-BSA
   . 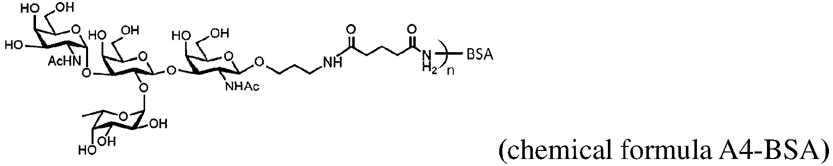
. A1-KLH
   . 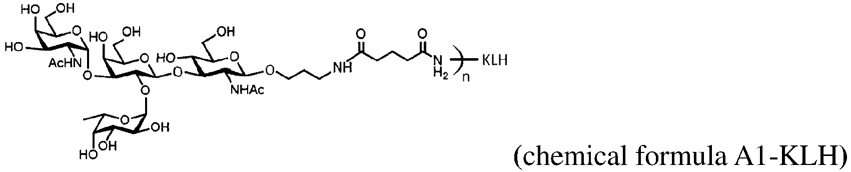
. A2-KLH
   . 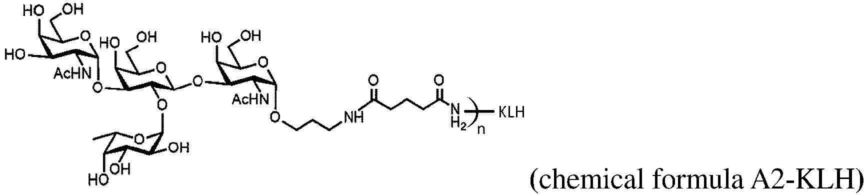
. A3-KLH
   . 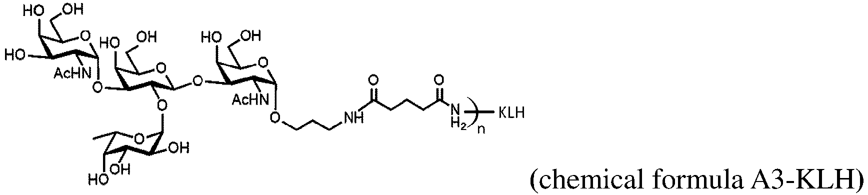
. A4-KLH
   . 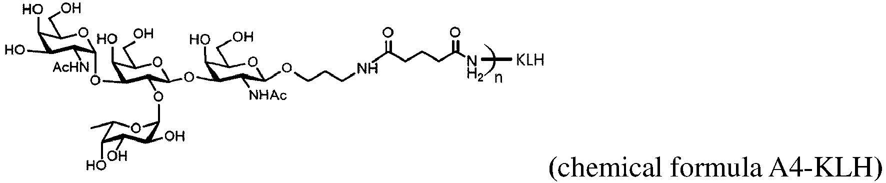
. wherein, n = 10, 20, 40, 60, 80, that is, a conjugation ratio of the blood group antigenic trisaccharide A analogue to the protein (the BSA or the KLH) is 10:1 - 80:1. See Table 1 for details.

### 1.2 Preparation of the blood group antigenic trisaccharide B analogue-protein conjugate

. First, four disaccharide precursors are synthesized with a strategy of chemically introducing a linker arm comprising an azide group at reducing ends of starting monosaccharides (GlcNAc, GlaNAc) and then taking the chemically synthesized GlcNAcβProN₃, GalNAcαProN₃ and GalNAcβProN₃ monosaccharides as acceptors to synthesize four type precursors by one-pot multienzyme. Chemical formulas thereof are as follows:

. Disaccharide precursor type I of the blood group antigenic trisaccharide B analogue:
.

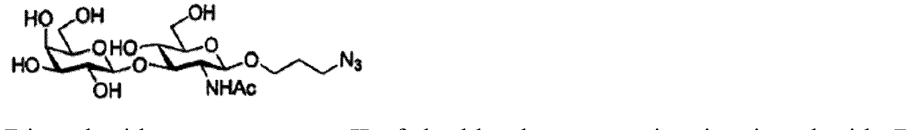

. Disaccharide precursor type II of the blood group antigenic trisaccharide B analogue:
.

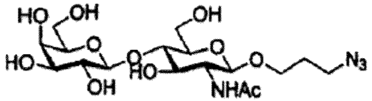

. Disaccharide precursor type III of the blood group antigenic trisaccharide B analogue:
.

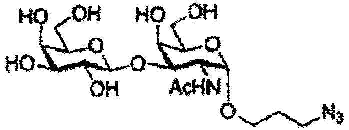

. Disaccharide precursor type IV of the blood group antigenic trisaccharide B analogue:
.

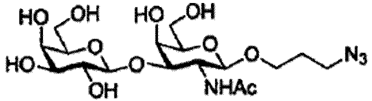

. Then, an α1-3 galactosyltransferase GTB is introduced at non-reducing ends of the above four synthesized disaccharide precursors to synthesize four blood group antigenic trisaccharide B analogues B1-B4 (molecular structures as shown in chemical formulas B1-B4), with specific synthesis routes as shown in Figs. 25-28.

. Finally, a linear linker arm with low immunogenicity, good reactivity and a high conjugation ratio is used to conjugate the blood group antigenic trisaccharide B analogues (B1-B4) to an amino group on a lysine residue of the BSA or the KLH (for example, a section of alkane chain connected with an azide group is pre-loaded on a starting substrate during synthesis of the sugar chain, as the linker arm, and then a reagent that make both ends of the linker arm form activated esters is used to achieve the conjugation of the blood group antigenic trisaccharide analogue to a carrier protein), thereby synthesizing the blood group antigenic trisaccharide B analogue-protein conjugates (B1-BSA, B2-BSA, B3-BSA, B4-BSA; B1-KLH, B2-KLH, B3-KLH, B4-KLH) of the present invention with chemical formulas shown as follows:
. B1-BSA
   . 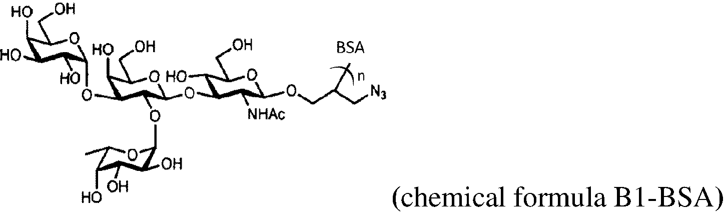
. B2-BSA
   . 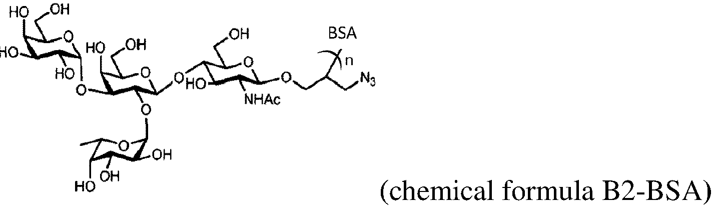
. B3-BSA
   . 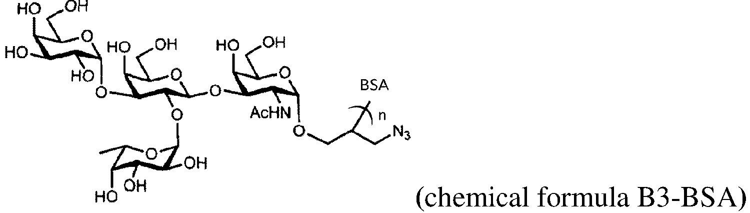
. B4-BSA
   . 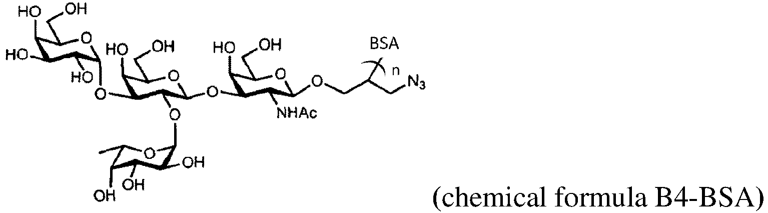
. B1-KLH
   . 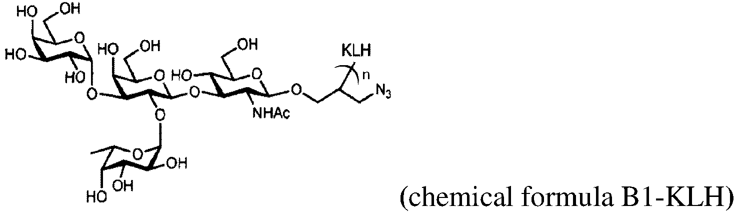
. B2-KLH
   . 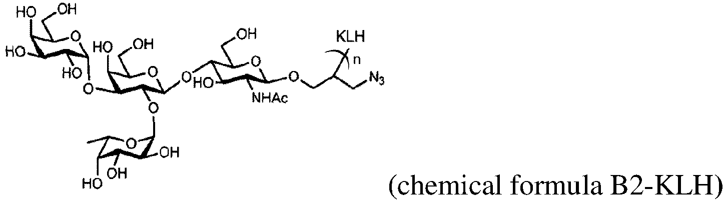
. B3-KLH
   . 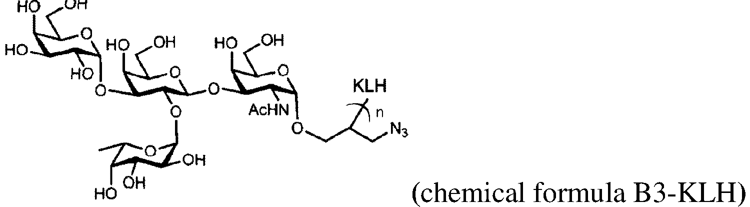
. B4-KLH
   . 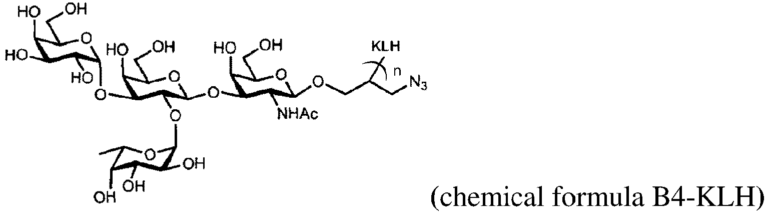
. wherein, n = 5, 10, 20, 40, that is, a conjugation ratio of the blood group antigenic trisaccharide B analogue to the KLH is 5:1-40:1. See Table 1 for details.

.

**Table 1 Conjugation ratios of the blood group antigenic trisaccharide analogue to the protein**

| B: BSA (conjugation ratio) | B: KLH (conjugation ratio) | A: BSA (conjugation ratio) | A: KLH (conjugation ratio) |
|---|---|---|---|
| B1:BSA=5:1 | B1:KLH=5:1 | A1:BSA=10:1 | A1:KLH=10:1 |
| B1:BSA=10:1 | B1:KLH=10:1 | A1:BSA=20:1 | A1:KLH=20:1 |
| B1:BSA=20:1 | B1:KLH=20:1 | A1:BSA=40:1 | A1:KLH=40:1 |
| B1:BSA=40:1 | B1:KLH=40:1 | A1:BSA=60:1 | A1:KLH=60:1 |
| B2:BSA=5:1 | B2:KLH=5:1 | A1:BSA=80:1 | A1:KLH=80:1 |
| B2:BSA=10:1 | B2:KLH=10:1 | A2:BSA=10:1 | A2:KLH=10:1 |
| B2:BSA=20:1 | B2:KLH=20:1 | A2:BSA=20:1 | A2:KLH=20:1 |
| B2:BSA=40:1 | B2:KLH=40:1 | A2:BSA=40:1 | A2:KLH=40:1 |
| B3:BSA=5:1 | B3:KLH=5:1 | A2:BSA=60:1 | A2:KLH=60:1 |
| B3:BSA=10:1 | B3:KLH=10:1 | A2:BSA=80:1 | A2:KLH=80:1 |
| B3:BSA=20:1 | B3:KLH=20:1 | A3:BSA=10:1 | A3:KLH=10:1 |
| B3:BSA=40:1 | B3:KLH=40:1 | A3:BSA=20:1 | A3:KLH=20:1 |
| B4:BSA=5:1 | B4:KLH=5:1 | A3:BSA=40:1 | A3:KLH=40:1 |
| B4:BSA=10:1 | B4:KLH=10:1 | A3:BSA=60:1 | A3:KLH=60:1 |
| B4:BSA=20:1 | B4:KLH=20:1 | A3:BSA=80:1 | A3:KLH=80:1 |
| B4:BSA=40:1 | B4:KLH=40:1 | A4:BSA=10:1 | A4:KLH=10:1 |
| / | / | A4:BSA=20:1 | A4:KLH=20:1 |
| / | / | A4:BSA=40:1 | A4:KLH=40:1 |
| / | / | A4:BSA=60:1 | A4:KLH=60:1 |
| / | / | A4:BSA=80:1 | A4:KLH=80:1 |

### . Embodiment 2

### . Qualitative verification of the blood group antigenic trisaccharide analogue-protein conjugate (antigen immobilization and immunochromatography)

. In order to investigate whether the blood group antigenic trisaccharide analogue-protein conjugates synthesized by the present invention can be used for simple and rapid qualitative blood grouping, the antigen immobilization and immunochromatography are used to perform verification in the present invention. In order to detect antibodies A and B in the serum, a detection card assembled from a Sartorius nitrocellulose (NC) membrane CN140 is used (see Figs. 1-8), wherein the NC membrane includes a test line (i.e. T line) and a control line (i.e. C line). The T line is immobilized and coated with the blood group antigenic trisaccharide A analogue-protein conjugate or the blood group antigenic trisaccharide B analogue-protein conjugate, respectively, and the C line is coated with an anti-chicken IgY antibody. A colloidal gold/microsphere pad comprises a labeled anti-human µ chain antibody and a labeled chicken IgY antibody.

. During detection, a sample was added from a sample hole (labeled as S on the detection card). The blood group antibody A or B comprised in the sample could be bound with the colloidal gold-labeled anti-human µ chain antibody to form a complex and chromatograph the complex on the NC membrane. When the complex was chromatographed to the T line, the blood group antibody A or B was specifically bound with the corresponding blood group antigenic trisaccharide A analogue-protein conjugate or blood group antigenic trisaccharide B analogue-protein conjugate to form a colored line that was visible with naked eyes (that is, the blood group antibody A was bound with an artificially synthesized antigen A, and the blood group antibody B was bound with an artificially synthesized antigen B). If there was no corresponding antibody A or B in the sample, the complex could not be formed and the color could not be visible at the T line. Based on this, when the sample comprised the antibody A and did not comprise the antibody B, the sample was group A blood. When the sample comprised the antibody B and did not comprise the antibody A, the sample was group B blood. When the sample comprised neither the antibody A nor the antibody B, the sample was group AB blood. When the sample comprised both the antibody A and the antibody B, the sample was group O blood. The sample was first chromatographed to the T line and then to the C line. The C line should be visible when all samples were detected, otherwise the experiment was invalid.

. For the detection card in the present application, the detection card coated with the blood group antigenic trisaccharide A analogue-protein conjugates (A-BSA, A-KLH) is referred to as a detection card A in the present invention. The detection card coated with the blood group antigenic trisaccharide B analogue-protein conjugates (B-BSA, B-KLH) is referred to as a detection card B in the present invention.

. 1 µl of a standard blood group antibody A (Millipore, batch No.: JHE2103 and catalog No.: JH-1L-BK) was added onto each detection card A, respectively, and then 80 µl of a sample diluent (0.01M PBS) was added, to observe the color development results at the C line and the T line with naked eyes after standing for about 15 min-20 min, and photograph and record.

. 1 µl of a standard blood group antibody B (Millipore, batch No.: JMC2103 and catalog No.: JM-1L-BK) was added onto each detection card B, respectively, then 80 µl of the sample diluent (0.01M PBS) was added, to observe the color development results at the C line and the T line with naked eyes after standing for about 15 min-20 min, and photograph and record.

. The above detection results were shown in Figs. 1-8, and summarized in Tables 2a-2d. Color development was indicated by "+" when it was visible with naked eyes, and indicated by "-" when it was invisible with naked eyes.

. The above detection results showed that for the blood group antigenic trisaccharide B analogue, the color could be effectively developed at the T line (visible with naked eyes) after the type B2 was conjugated to the BSA or the KLH. However, the color development effect of B2-BSA was obvious only when a conjugation ratio was 20:1, while B2-KLH showed a better color development effect in a wider conjugation ratio range (from 5:1 to 40:1). In addition, when the conjugation ratio of B-KLH was 40:1, color development effects of B1-KLH, B3-KLH and B4-KLH could also be visible with naked eyes. For the blood group antigenic trisaccharide A analogue, whether A2-A4 were conjugated to the BSA or the KLH, the formed blood group antigenic trisaccharide protein conjugates could effectively develop color at the T line (conjugation ratios: 10:1, 40:1, 60:1, 80:1). However, when the conjugation ratios of A-BSA and A-KLH were 20:1, only A2-BSA/A2-KLH could effectively develop color, and neither of A3-BSA/A3-KLH and A4-BSA/A4-KLH could effectively develop color. Moreover, whether A1 was conjugated to the BSA or the KLH, the color could not be effectively developed at the conjugation ratios tested in the present invention.

. The above experimental results showed that after a specific blood group antigenic trisaccharide analogue was conjugated to a specific protein (for example, B2-KLH, A2-BSA, A2-KLH), or at a specific conjugation ratio (20:1 (B2-BSA); 40:1 (B1-KLH, B3-KLH, B4-KLH); 10:1, 40:1-80:1 (A3-BSA, A4-BSA, A3-KLH, A4-KLH)), when the standard blood group antibodies were detected, the color could be effectively developed at the T line and was visible with naked eyes, which proved that these blood group antigenic trisaccharide analogues could be stably and effectively immobilized on the NC membrane or other types of solid-phase media (for example, ELISA plates, various materials of microspheres or magnetic beads) after protein conjugation, and could effectively and specifically capture the corresponding blood group antibodies. Therefore, the blood group antigenic trisaccharide analogues can be used for rapid and easy qualitative detection of the blood group antibodies (for example, due to visibility with naked eyes, the results can be read by ordinary people), so that blood grouping can be completed at home or in small clinics.

. For those skilled in the art, the blood group antigenic trisaccharide analogue-protein conjugates of the present invention can also be used for experimental systems using other labeling methods (for example, enzyme labeling, fluorescence labeling or other illuminant labeling). Furthermore, in addition to labeling the antibodies, the blood group antigenic trisaccharide analogue-protein conjugates can also be labeled by different labeling methods, and used for various heterogeneous or homogeneous detection according to actual needs.

.

**Table 2a**

| Detected samples | Antibody A | | | | Antibody B | | | |
|---|---|---|---|---|---|---|---|---|
| Conjugation ratios | 5:1 | 10:1 | 20:1 | 40:1 | 5:1 | 10:1 | 20:1 | 40:1 |
| B1-BSA | - | - | - | - | - | - | - | - |
| B2-BSA | - | - | - | - | - | - | + | - |
| B3-BSA | - | - | - | - | - | - | - | - |
| B4-BSA | - | - | - | - | - | - | - | - |

.

**Table 2b**

| Detected samples | Antibody A | | | | Antibody B | | | |
|---|---|---|---|---|---|---|---|---|
| Conjugation ratios | 5:1 | 10:1 | 20:1 | 40:1 | 5:1 | 10:1 | 20:1 | 40:1 |
| B1-KLH | - | - | - | - | - | - | - | + |
| B2-KLH | - | - | - | - | + | + | + | + |
| B3-KLH | - | - | - | - | - | - | - | + |
| B4-KLH | - | - | - | - | - | - | - | + |

.

**Table 2c**

| Detected samples | Antibody A | | | | | Antibody B | | | | |
|---|---|---|---|---|---|---|---|---|---|---|
| Conjugation ratios | 10:1 | 20:1 | 40:1 | 60:1 | 80:1 | 10:1 | 20:1 | 40:1 | 60:1 | 80:1 |
| A1-BSA | - | - | - | - | - | - | - | - | - | - |
| A2-BSA | + | + | + | + | + | - | - | - | - | - |
| A3-BSA | + | - | + | + | + | - | - | - | - | - |
| A4-BSA | + | - | + | + | + | - | - | - | - | - |

.

**Table 2d**

| Detected samples | Antibody A | | | | | Antibody B | | | | |
|---|---|---|---|---|---|---|---|---|---|---|
| Conjugation ratios | 10:1 | 20:1 | 40:1 | 60:1 | 80:1 | 10:1 | 20:1 | 40:1 | 60:1 | 80:1 |
| A1-KLH | - | - | - | - | - | - | - | - | - | - |
| A2-KLH | + | + | + | + | + | - | - | - | - | - |
| A3-KLH | + | - | + | + | + | - | - | - | - | - |
| A4-KLH | + | - | + | + | + | - | - | - | - | - |

### . Embodiment 3

### . Qualitative verification of the blood group antigenic trisaccharide B analogue-protein conjugate

### . 3.1 Experiment on conjugation of the blood group antigenic trisaccharide B to the KLH

### . 1) Detection results on diluting standard antibodies by 100 times

. Main experiment reagents used in the experiment included latex beads (125 µL of the latex beads, labeling a total of 0.1 mg of the antigen) and a reagent R1 (1 g BSA, 0.24 g Tris, 0.1 g PC300, 5 g PEG6000, 100 mL of water, pH 8.5).

. The loading method of the experiment was as follows: first standard antibodies A and B (Millipore, batch No.: JHE2103 and catalog No.: JH-1L-BK; Millipore, batch No.: JMC2103 and catalog No.: JM-1L-BK) were diluted with R1 by 100 times to obtain the diluted standard antibodies A and B with a volume of 200 µL, and then 200 µL of a reagent R2 (that is, the above latex beads were diluted with TBS by 100 times) was added to obtain a total of 400 µL of the sample to be detected. In the experiment, a detection wavelength was 340 nm.

. The above experimental results were summarized in the following table:

.

**Table 3a**

| Conjugation ratios | OD value of antibody A | OD value of antibody B | P/N |
|---|---|---|---|
| B1:KLH=5:1 | 0.251 | 0.362 | 1.442 |
| B1:KLH=10:1 | 0.239 | 0.338 | 1.416 |
| B1:KLH=20:1 | 0.253 | 0.384 | 1.519 |
| B1:KLH=40:1 | 0.257 | 0.417 | 1.622 |
| B2:KLH=5:1 | 0.237 | 0.367 | 1.547 |
| B2:KLH=10:1 | 0.236 | 0.405 | 1.715 |
| B2:KLH=20:1 | 0.261 | 0.642 | 2.459 |
| B2:KLH=40:1 | 0.227 | 0.424 | 1.869 |
| B3:KLH=5:1 | 0.243 | 0.350 | 1.442 |
| B3:KLH=10:1 | 0.229 | 0.343 | 1.498 |
| B3:KLH=20:1 | 0.234 | 0.334 | 1.427 |
| B3:KLH=40:1 | 0.251 | 0.405 | 1.614 |
| B4:KLH=5:1 | 0.237 | 0.342 | 1.441 |
| B4:KLH=10:1 | 0.242 | 0.362 | 1.497 |
| B4:KLH=20:1 | 0.248 | 0.374 | 1.508 |
| B4:KLH=40:1 | 0.252 | 0.410 | 1.627 |

. The above experimental results showed that after the antigen B used in the present invention was conjugated to the KLH, regardless of the type from B1-B4, and regardless of the conjugation ratio to the KLH in a range of 5:1-40:1, the OD value (greater than or equal to 0.334) of the antigen B bound with the antibody B was apparently higher than the OD value (the highest OD value only could reach 0.261) of the antigen B bound with the antibody A, and the P/ N value (OD value of positive serum/OD value of negative serum) could reach 1.4 or more, that is, the antigen B used in the present invention could well distinguish the antibodies A and B in the detection of the blood group antibodies and had better specificity. It was worth noting that for B1-KLH, B3-KLH and B4-KLH, when the conjugation ratio was 40:1, the P/N value reached the maximum, that is, the antigen B could distinguish the antibodies A and B most. The P/N value of B2-KLH was relatively high in the conjugation ratio range (5:1-40:1) used in the present invention, and especially when the conjugation ratio was 20:1, the P/N value reached the maximum, up to 2.459.

### . 2) Detection results on diluting standard antibodies by 200 times

. In order to further verify the detection effect of the blood group antigenic trisaccharide B analogue-protein conjugate (B-KLH) synthesized by the present invention when the antibody concentration was lower, the standard antibodies were diluted by 200 times in the experiment. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5). The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 200 times to obtain the diluted standard antibodies with a volume of 200 µL, and then 200 µL of the reagent R2 (that is, the above latex beads were diluted with TBS by 200 times) was added to obtain a total of 400 µL of the sample to be detected. In the experiment, a detection wavelength was 340 nm.

. The above experimental results were summarized in the following table:

.

**Table 3b**

| Conjugation ratios | OD value of antibody A | OD value of antibody B | P/N |
|---|---|---|---|
| B1:KLH=5:1 | 0.251 | 0.307 | 1.224 |
| B1:KLH=10:1 | 0.239 | 0.319 | 1.336 |
| B1:KLH=20:1 | 0.253 | 0.347 | 1.371 |
| B1:KLH=40:1 | 0.257 | 0.379 | 1.475 |
| B2:KLH=5:1 | 0.237 | 0.346 | 1.458 |
| B2:KLH=10:1 | 0.236 | 0.383 | 1.624 |
| B2:KLH=20:1 | 0.261 | 0.557 | 2.133 |
| B2:KLH=40:1 | 0.227 | 0.403 | 1.777 |
| B3:KLH=5:1 | 0.243 | 0.311 | 1.281 |
| B3:KLH=10:1 | 0.229 | 0.300 | 1.311 |
| B3:KLH=20:1 | 0.234 | 0.316 | 1.352 |
| B3:KLH=40:1 | 0.251 | 0.367 | 1.462 |
| B4:KLH=5:1 | 0.237 | 0.330 | 1.393 |
| B4:KLH=10:1 | 0.242 | 0.322 | 1.329 |
| B4:KLH=20:1 | 0.248 | 0.361 | 1.456 |
| B4:KLH=40:1 | 0.252 | 0.373 | 1.481 |

. The above experimental results showed that even if the standard antibodies were diluted by 200 times, for the antigen B used in the present invention, regardless of the type from B1-B4, and regardless of the conjugation ratio to the KLH in a range of 5:1-40:1, the OD value (greater than or equal to 0.300) of the antigen B bound with the antibody B was apparently higher than the OD value (the highest OD value only could reach 0.261) of the antigen B bound with the antibody A, and the P/N value could almost reach 1.3 or more (except for B1-KLH and B3-KLH when the conjugation ratio was 5:1). In other words, even if the antibody concentration was lower, the antigen B used in the present invention could well distinguish the antibodies A and B in the detection of the blood group antibodies.

### . 3.2 Experiment on conjugation the blood group antigenic trisaccharide B to the BSA

### . 1) Detection results on diluting standard antibodies by 100 times

. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5).

. The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 100 times to obtain the diluted standard antibodies with a volume of 200 µL, and then 200 µL of the reagent R2 (that is, the above latex beads were diluted with TBS by 100 times) was added to obtain a total of 400 µL of the sample to be detected. In the experiment, a detection wavelength was 340 nm.

. The above experimental results were summarized in the following table:

.

**Table 3c**

| Conjugation ratios | OD value of antibody A | OD value of antibody B | P/N |
|---|---|---|---|
| B1:BSA=5:1 | 0.187 | 0.233 | 1.244 |
| B1:BSA=10:1 | 0.188 | 0.241 | 1.279 |
| B1:BSA=20:1 | 0.176 | 0.254 | 1.446 |
| B1:BSA=40:1 | 0.182 | 0.239 | 1.314 |
| B2:BSA=5:1 | 0.177 | 0.258 | 1.459 |
| B2:BSA=10:1 | 0.161 | 0.239 | 1.483 |
| B2:BSA=20:1 | 0.147 | 0.247 | 1.678 |
| B2:BSA=40:1 | 0.171 | 0.261 | 1.522 |
| B3:BSA=5:1 | 0.185 | 0.228 | 1.231 |
| B3:BSA=10:1 | 0.200 | 0.253 | 1.267 |
| B3:BSA=20:1 | 0.162 | 0.244 | 1.503 |
| B3:BSA=40:1 | 0.180 | 0.238 | 1.325 |
| B4:BSA=5:1 | 0.187 | 0.256 | 1.367 |
| B4:BSA=10:1 | 0.177 | 0.239 | 1.352 |
| B4:BSA=20:1 | 0.194 | 0.253 | 1.303 |
| B4:BSA=40:1 | 0.181 | 0.262 | 1.451 |

. The above experimental results showed that for the antigen B used in the present invention, regardless of the type from B1-B4, and regardless of the conjugation ratio to the BSA in a range of 5:1-40:1, although there was a difference between the OD value of the antigen B bound with the antibody B and the OD value of the antigen B bound with the antibody A, the difference was not as good as the difference between the OD values when the antigen B was conjugated to the KLH to identify the antibodies A and B. The P/N value in multiple sets of data was in a range of 1.231-1.678. The above results proved that the conjugate of the blood group antigenic trisaccharide B (B1-B4) analogue to the BSA could also detect the antibody B and distinguish the antibodies A and B in the above experiment, but the ability and effect thereof were not as good as those when the blood group antigenic trisaccharide B (B1-B4) analogue was conjugated to the KLH.

### . 2) Detection results on diluting standard antibodies by 200 times

. In order to further verify the detection effect of the blood group antigenic trisaccharide B analogue-protein conjugate (B-BSA) synthesized by the present invention when the antibody concentration was lower, the standard antibodies were diluted by 200 times in the experiment. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5). The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 200 times to obtain the diluted standard antibodies with a volume of 200 µL, and then 200 µL of the reagent R2 (that is, the above latex beads were diluted with TBS by 200 times) was added to obtain a total of 400 µL of the sample to be detected. In the experiment, a detection wavelength was 340 nm.

. The above experimental results were summarized in the following table:

.

**Table 3d**

| Conjugation ratios | OD value of antibody A | OD value of antibody B | P/N |
|---|---|---|---|
| B1:BSA=5:1 | 0.243 | 0.295 | 1.214 |
| B1:BSA=10:1 | 0.239 | 0.292 | 1.223 |
| B1:BSA=20:1 | 0.253 | 0.353 | 1.396 |
| B1:BSA=40:1 | 0.244 | 0.311 | 1.276 |
| B2:BSA=5:1 | 0.233 | 0.321 | 1.376 |
| B2:BSA=10:1 | 0.229 | 0.319 | 1.392 |
| B2:BSA=20:1 | 0.252 | 0.358 | 1.421 |
| B2:BSA=40:1 | 0.254 | 0.337 | 1.325 |
| B3:BSA=5:1 | 0.228 | 0.274 | 1.201 |
| B3:BSA=10:1 | 0.237 | 0.277 | 1.168 |
| B3:BSA=20:1 | 0.245 | 0.342 | 1.394 |
| B3:BSA=40:1 | 0.257 | 0.316 | 1.231 |
| B4:BSA=5:1 | 0.251 | 0.304 | 1.211 |
| B4:BSA=10:1 | 0.243 | 0.309 | 1.272 |
| B4:BSA=20:1 | 0.231 | 0.280 | 1.214 |
| B4:BSA=40:1 | 0.229 | 0.297 | 1.296 |

. The above experimental results were similar to the detection results on diluting the standard antibodies by 100 times. Regardless of the type from B1-B4, and regardless of the conjugation ratio to the BSA in a range of 5:1-40:1, although there was a difference between the OD value of the antigen B bound with the antibody B and the OD value of the antigen B bound with the antibody A, the difference was not as good as the difference between the OD values when the antigen B was conjugated to the KLH to identify the antibodies A and B. Only when B2:BSA=20:1, the P/N value could reach 1.421, and when B3:BSA=20:1, the P/N value could reach 1.394. In combination with the color development experiment on the NC membrane (Table 2a), only when B2:BSA=20:1, a chromogenic reaction could be observed on the detection card with naked eyes. The above results proved that the conjugate of the blood group antigenic trisaccharide B (B1-B4) analogue to the BSA could also detect the antibody B and distinguish the antibodies A and B to some extent, but the ability thereof was significantly inferior to that when the blood group antigenic trisaccharide B (B1-B4) analogue was conjugated to the KLH.

### . Embodiment 4

### . Qualitative verification of the blood group antigenic trisaccharide A analogue-protein conjugate (optical density experiment)

### . 4.1 Experiment on conjugation of the blood group antigenic trisaccharide A to the BSA

### . 1) Detection results on diluting standard antibodies by 100 times

. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5).

. The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 100 times to obtain the diluted standard antibodies with a volume of 200 µL, and then 200 µL of the reagent R2 (that is, the above latex beads were diluted with TBS by 100 times) was added to obtain a total of 400 µL of the sample to be detected. In the experiment, a detection wavelength was 340 nm.

. The above experimental results were summarized in the following table:

.

**Table 4a**

| Conjugation ratios | OD value of antibody A | OD value of antibody B | P/N |
|---|---|---|---|
| A1:BSA=10:1 | 0.281 | 0.235 | 1.194 |
| A1:BSA=20:1 | 0.284 | 0.244 | 1.163 |
| A1:BSA=40:1 | 0.312 | 0.239 | 1.305 |
| A1:BSA=60:1 | 0.307 | 0.251 | 1.223 |
| A1:BSA=80:1 | 0.281 | 0.253 | 1.109 |
| A2:BSA=10:1 | 0.420 | 0.228 | 1.844 |
| A2:BSA=20:1 | 0.453 | 0.231 | 1.959 |
| A2:BSA=40:1 | 0.619 | 0.224 | 2.764 |
| A2:BSA=60:1 | 0.428 | 0.236 | 1.813 |
| A2:BSA=80:1 | 0.407 | 0.225 | 1.809 |
| A3:BSA=10:1 | 0.416 | 0.243 | 1.711 |
| A3:BSA=20:1 | 0.290 | 0.239 | 1.212 |
| A3:BSA=40:1 | 0.407 | 0.241 | 1.688 |
| A3:BSA=60:1 | 0.410 | 0.242 | 1.696 |
| A3:BSA=80:1 | 0.392 | 0.227 | 1.725 |
| A4:BSA=10:1 | 0.415 | 0.238 | 1.744 |
| A4:BSA=20:1 | 0.364 | 0.244 | 1.491 |
| A4:BSA=40:1 | 0.408 | 0.237 | 1.723 |
| A4:BSA=60:1 | 0.432 | 0.245 | 1.764 |
| A4:BSA=80:1 | 0.404 | 0.237 | 1.705 |

. The above experimental results showed that when the antigen A used in the present invention was conjugated to the BSA, regardless of the conjugation ratio of most of A2-A4 to the BSA in a range of 10:1-80:1 (except for A3:BSA=20:1, A4:BSA=20:1), the OD value (greater than or equal to 0.4) of the antigen A bound with the antibody A was higher than the OD value (the highest OD value only could reach 0.245) of the antigen A bound with the antibody B. The experiment proved that the antigens A2-A4 used in the present invention could well distinguish the antibodies A and B in the detection of the blood group antibodies, especially the distinguishing effect was the best when A2:BSA=40:1. By comparison, the A1-BSA conjugate had a poorer ability to identify the antibodies A and B, and the P/N value was only slightly higher than 1.

### . 2) Detection results on diluting standard antibodies by 200 times

. In order to further verify the detection effect of the blood group antigenic trisaccharide A analogue-protein conjugate (A-BSA) synthesized by the present invention when the antibody concentration was lower, the standard antibodies were diluted by 200 times in the experiment. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5). The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 200 times to obtain the diluted standard antibodies with a volume of 200 µL, and then 200 µL of the reagent R2 (that is, the above latex beads were diluted with TBS by 200 times) was added to obtain a total of 400 µL of the sample to be detected. In the experiment, a detection wavelength was 340 nm.

. The above experimental results were summarized in the following table:

.

**Table 4b**

| Conjugation ratios | OD value of antibody A | OD value of antibody B | P/N |
|---|---|---|---|
| A1:BSA=10:1 | 0.273 | 0.235 | 1.161 |
| A1:BSA=20:1 | 0.269 | 0.244 | 1.102 |
| A1:BSA=40:1 | 0.304 | 0.239 | 1.271 |
| A1:BSA=60:1 | 0.282 | 0.251 | 1.125 |
| A1:BSA=80:1 | 0.277 | 0.253 | 1.093 |
| A2:BSA=10:1 | 0.382 | 0.228 | 1.674 |
| A2:BSA=20:1 | 0.396 | 0.231 | 1.715 |
| A2:BSA=40:1 | 0.575 | 0.224 | 2.569 |
| A2:BSA=60:1 | 0.400 | 0.236 | 1.694 |
| A2:BSA=80:1 | 0.387 | 0.225 | 1.718 |
| A3:BSA=10:1 | 0.394 | 0.243 | 1.621 |
| A3:BSA=20:1 | 0.267 | 0.239 | 1.119 |
| A3:BSA=40:1 | 0.368 | 0.241 | 1.527 |
| A3:BSA=60:1 | 0.365 | 0.242 | 1.508 |
| A3:BSA=80:1 | 0.368 | 0.227 | 1.622 |
| A4:BSA=10:1 | 0.375 | 0.238 | 1.577 |
| A4:BSA=20:1 | 0.316 | 0.244 | 1.296 |
| A4:BSA=40:1 | 0.381 | 0.237 | 1.609 |
| A4:BSA=60:1 | 0.397 | 0.245 | 1.622 |
| A4:BSA=80:1 | 0.365 | 0.237 | 1.538 |

. The above experimental results showed that even if the standard samples were diluted by 200 times and the antibody concentration was low, when the antigens A2-A4 used in the present invention were conjugated to the BSA, regardless of the conjugation ratio to the BSA in a range of 10:1-80:1 (except for A3:BSA=20:1, A4:BSA=20:1), the P/N value was relatively high, that is, the antigens A2-A4 could still distinguish the antibodies A and B well in the detection of the blood group antibodies, especially the distinguishing effect was the best when A2:BSA=40:1. By comparison, the A1-BSA conjugate had a poorer ability to identify the antibodies A and B, and the P/N value was only slightly higher than 1.

### . 4.2 Experiment on conjugation of the blood group antigenic trisaccharide A to the KLH

### . 1) Detection results on diluting standard antibodies by 100 times

. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5).

. The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 100 times to obtain the diluted standard antibodies with a volume of 200 µL, and then 200 µL of the reagent R2 (that is, the above latex beads were diluted with TBS by 100 times) was added to obtain a total of 400 µL of the sample to be detected. In the experiment, a detection wavelength was 340 nm.

. The above experimental results were summarized in the following table:

.

**Table 4c**

| Conjugation ratios | OD value of antibody A | OD value of antibody B | P/N |
|---|---|---|---|
| A1:KLH=10:1 | 0.284 | 0.255 | 1.112 |
| A1:KLH=20:1 | 0.342 | 0.264 | 1.297 |
| A1:KLH=40:1 | 0.346 | 0.259 | 1.337 |
| A1:KLH=60:1 | 0.317 | 0.251 | 1.263 |
| A1:KLH=80:1 | 0.342 | 0.253 | 1.351 |
| A2:KLH=10:1 | 0.479 | 0.266 | 1.801 |
| A2:KLH=20:1 | 0.524 | 0.271 | 1.933 |
| A2:KLH=40:1 | 0.629 | 0.283 | 2.223 |
| A2:KLH=60:1 | 0.489 | 0.264 | 1.854 |
| A2:KLH=80:1 | 0.489 | 0.269 | 1.819 |
| A3:KLH=10:1 | 0.485 | 0.277 | 1.751 |
| A3:KLH=20:1 | 0.494 | 0.283 | 1.744 |
| A3:KLH=40:1 | 0.485 | 0.274 | 1.769 |
| A3:KLH=60:1 | 0.464 | 0.285 | 1.627 |
| A3:KLH=80:1 | 0.473 | 0.293 | 1.613 |
| A4:KLH=10:1 | 0.473 | 0.288 | 1.641 |
| A4:KLH=20:1 | 0.459 | 0.271 | 1.694 |
| A4:KLH=40:1 | 0.484 | 0.284 | 1.704 |
| A4:KLH=60:1 | 0.474 | 0.275 | 1.723 |
| A4:KLH=80:1 | 0.472 | 0.272 | 1.737 |

. The above experimental results showed that when the antigen A used in the present invention was conjugated to the KLH, regardless of the conjugation ratio of most of A2-A4 to the KLH in a range of 10:1-80:1, the OD value (greater than or equal to 0.4) of the antigen A bound with the antibody A was higher than the OD value (the highest OD value only could reach 0.293) of the antigen A bound with the antibody B. The experiment proved that the antigens A2-A4 used in the present invention could well distinguish the antibodies A and B in the detection of the blood group antibodies, especially the distinguishing effect was the best when A2:KLH=40:1. By comparison, the A1-KLH conjugate had a poorer ability to identify the antibodies A and B, and the P/N value was only slightly higher than 1.

### . 2) Detection results on diluting standard antibodies by 200 times

. In order to further verify the detection effect of the blood group antigenic trisaccharide A analogue-protein conjugate (A-KLH) synthesized by the present invention when the antibody concentration was lower, the standard antibodies were diluted by 200 times in the experiment. Main experiment reagents used in the experiment included latex beads (125 µL of the latex beads, labeling a total of 0.1 mg of the antigen) and the reagent R1 (1 g BSA, 0.24 g Tris, 0.1 g PC300, 5 g PEG6000, 100 mL of water, pH 8.5). The loading method of the experiment was as follows: standard antibodies A and B (Millipore, batch No.: JHE2103 and catalog No.: JH-1L-BK; Millipore, batch No.: JMC2103 and catalog No.: JM-1L-BK) were diluted with R1 by 200 times to obtain the diluted standard antibodies with a volume of 200 µL, and then 200 µL of the reagent R2 (that is, the above latex beads were diluted with TBS by 200 times) was added to obtain a total of 400 µL of the sample to be detected. In the experiment, a detection wavelength was 340 nm.

. The above experimental results were summarized in the following table:

.

**Table 4d**

| Conjugation ratios | OD value of antibody A | OD value of antibody B | P/N |
|---|---|---|---|
| A1:KLH=10:1 | 0.262 | 0.255 | 1.029 |
| A1:KLH=20:1 | 0.324 | 0.264 | 1.227 |
| A1:KLH=40:1 | 0.331 | 0.259 | 1.278 |
| A1:KLH=60:1 | 0.287 | 0.251 | 1.145 |
| A1:KLH=80:1 | 0.308 | 0.253 | 1.217 |
| A2:KLH=10:1 | 0.447 | 0.266 | 1.679 |
| A2:KLH=20:1 | 0.479 | 0.271 | 1.766 |
| A2:KLH=40:1 | 0.614 | 0.283 | 2.170 |
| A2:KLH=60:1 | 0.447 | 0.264 | 1.694 |
| A2:KLH=80:1 | 0.446 | 0.269 | 1.657 |
| A3:KLH=10:1 | 0.443 | 0.277 | 1.601 |
| A3:KLH=20:1 | 0.449 | 0.283 | 1.585 |
| A3:KLH=40:1 | 0.435 | 0.274 | 1.588 |
| A3:KLH=60:1 | 0.416 | 0.285 | 1.461 |
| A3:KLH=80:1 | 0.439 | 0.293 | 1.498 |
| A4:KLH=10:1 | 0.422 | 0.288 | 1.464 |
| A4:KLH=20:1 | 0.407 | 0.271 | 1.503 |
| A4:KLH=40:1 | 0.431 | 0.284 | 1.519 |
| A4:KLH=60:1 | 0.420 | 0.275 | 1.527 |
| A4:KLH=80:1 | 0.414 | 0.272 | 1.523 |

. The above experimental results showed that even if the standard samples were diluted by 200 times, when the antigens A2-A4 used in the present invention were conjugated to the KLH, regardless of the conjugation ratio to the KLH in a range of 10:1-80:1, the P/N value was relatively higher, that is, the antigens A2-A4 could still distinguish the antibodies A and B well in the detection of the blood group antibodies, especially the distinguishing effect was the best when A2:KLH=40:1. By comparison, the A1-KLH conjugate had a poorer ability to identify the antibodies A and B, and the P/N value was only slightly higher than 1.

### . Embodiment 5

### . Neutralization experiment of the blood group antigenic trisaccharide analogue-protein conjugate using column agglutination

. A reverse human ABO grouping detection card (column agglutination) was used in the embodiment to perform a neutralization experiment, that is, first an artificial antigen was used to neutralize a certain amount of an antibody, and then indicator cells were added to check whether there was still the antibody that had not yet been neutralized by the artificial antigen. If the artificial antigen had strong specificity and high efficiency when bound with the antibody, the antibody could be fully neutralized by the artificial antigen, and when the indicator cells (corresponding to the antibody) were added, there was no antibody that could react with surface antigens of the indicator cells, so that the experimental result was negative (without agglutination). Conversely, if the artificial antigen had weak specificity and low efficiency when bound with the antibody, the antibody could not be fully neutralized by the artificial antigen, and when the indicator cells (corresponding to the antibody) were added, there was still the antibody that could react with surface antigens of the indicator cells, so that the experimental result was positive (with agglutination). Through the above experimental design, the performance of the reaction between the artificially synthesized antigens (that is, the blood group antigenic trisaccharide B analogue-protein conjugate and the blood group antigenic trisaccharide A analogue-protein conjugate according to the present invention) and the antibodies could be effectively verified, that is, whether the artificially synthesized antigen had good specificity in identifying its corresponding antibody.

. The information of the standard antibody A used in the experiment is: Millipore, batch No.: JHE2103 and catalog No.: JH-1L-BK. The information of the standard antibody B used in the experiment is: Millipore, batch No.: JMC2103 and catalog No.: JM-1L-BK.

### . 5.1 Detecting antibodies with the B-BSA antigen

. 1) First, the standard antibody B (Ab B) was diluted with normal saline (NS) by 10 times (10X), 100 times (100X), 1000 times (1KX), 10000 times (1WX) and 100000 times(10WX), respectively, and then the diluted antibodies (with normal saline as negative control) were mixed with an equal volume of an antigen B (0.01 mg/mL) with different structures and different BSA conjugation ratios and incubated at room temperature for about 15 min. Then 40 µL of the incubated mixture was added into the above reverse ABO grouping detection card as an object to be detected. Finally, 10 µL of indicator cells B (with the blood group antigen B) were added, and the results were read after centrifugation. The results were shown in the following table:
.

**Table 5a**

| | Dilution times of the standard antibody B (Ab B) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| B1:BSA=5:1 | + | + | + | + | - | - | Fig. 17 |
| B1:BSA=10:1 | + | + | + | + | - | - | |
| B1:BSA=20:1 | + | + | + | - | - | - | |
| B1:BSA=40:1 | + | + | + | + | - | - | |
| B2:BSA=5:1 | + | + | + | - | - | - | Fig. 18 |
| B2:BSA=10:1 | + | + | + | - | - | - | |
| B2:BSA=20:1 | + | + | - | - | - | - | |
| B2:BSA=40:1 | + | + | + | - | - | - | |
| B3:BSA=5:1 | + | + | + | + | - | - | Fig. 19 |
| B3:BSA=10:1 | + | + | + | + | - | - | |
| B3:BSA=20:1 | + | + | + | - | - | - | |
| B3:BSA=40:1 | + | + | + | + | - | - | |
| B4:BSA=5:1 | + | + | + | + | - | - | Fig. 20 |
| B4:BSA=10:1 | + | + | + | + | - | - | |
| B4:BSA=20:1 | + | + | + | + | - | - | |
| B4:BSA=40:1 | + | + | + | - | - | - | |

. 2) First, the standard antibody A (Ab A) was diluted with normal saline by 10 times, 100 times, 1000 times, 10000 times and 100000 times, respectively, and then the diluted antibodies (with normal saline as negative control) were mixed with an equal volume of an antigen B (0.01 mg/mL) with different structures and different BSA conjugation ratios and incubated at room temperature for about 15 min. 40 µL of the incubated mixture was added into the above reverse ABO grouping detection card as an object to be detected. Then, 10 µL of the indicator cells B (with the blood group antigen B) were added, and the results were read after centrifugation. The results were shown in the following table:

.

**Table 5b**

| | Dilution times of the standard antibody A (Ab A) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| B1:BSA=5:1 | - | - | - | - | - | - | Fig. 17 |
| B1:BSA=10:1 | - | - | - | - | - | - | |
| B1:BSA=20:1 | - | - | - | - | - | - | |
| B1:BSA=40:1 | - | - | - | - | - | - | |
| B2:BSA=5:1 | - | - | - | - | - | - | Fig. 18 |
| B2:BSA=10:1 | - | - | - | - | - | - | |
| B2:BSA=20:1 | - | - | - | - | - | - | |
| B2:BSA=40:1 | - | - | - | - | - | - | |
| B3:BSA=5:1 | - | - | - | - | - | - | Fig. 19 |
| B3:BSA=10:1 | - | - | - | - | - | - | |
| B3:BSA=20:1 | - | - | - | - | - | - | |
| B3:BSA=40:1 | - | - | - | - | - | - | |
| B4:BSA=5:1 | - | - | - | - | - | - | Fig. 20 |
| B4:BSA=10:1 | - | - | - | - | - | - | |
| B4:BSA=20:1 | - | - | - | - | - | - | |
| B4:BSA=40:1 | - | - | - | - | - | - | |

. Experimental results: Table 5a showed that the antigen B (B1-B4) conjugated to the BSA had generally weak neutralization ability for the antibody B. By comparison within B-BSA, B2-BSA was better (when the antibody B was diluted by 10000 times, the experimental results were negative at all conjugation ratios), especially the neutralization ability was relatively stronger when B2:BSA=20:1 (the antibody B could be fully neutralized when diluted by 1000 times). When the antibody B was diluted by 1000 times, B1-BSA and B3-BSA showed better neutralization abilities for the antibody B when the conjugation ratio was 20:1 when compared with the same type of antigens at other conjugation ratios. B4-BSA showed better neutralization ability for the antibody B when the conjugation ratio was 40:1 when compared with the same type of antigens at other conjugation ratios. Table 5b was a control experiment using the antibody A, in which the antigen B (B1-B4) conjugated to the BSA should neither perform a neutralization reaction nor react with the indicator cells B, so that the experimental results were all negative, proving that the experimental system operated normally.

### . 5.2 Detecting antibodies with the B-KLH antigen

. 1) First, the standard antibody B was diluted with normal saline by 10 times, 100 times, 1000 times, 10000 times and 100000 times, respectively, and then the diluted antibodies (with normal saline as negative control) were mixed with an equal volume of an antigen B (0.01 mg/mL) with different structures and different KLH conjugation ratios and incubated at room temperature for about 15 min. Then 40 µL of the incubated mixture was added into the above reverse ABO grouping detection card as an object to be detected. Finally, 10 µL of indicator cells B were added, and the results were read after centrifugation. The results were shown in the following table:
.

**Table 5c**

| | Dilution times of the standard antibody B (Ab B) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| B1:KLH=5:1 | + | + | + | - | - | - | Fig. 21 |
| B1:KLH=10:1 | + | + | + | - | - | - | |
| B1:KLH=20:1 | + | + | + | - | - | - | |
| B1:KLH=40:1 | + | + | - | - | - | - | |
| B2:KLH=5:1 | + | + | + | - | - | - | Fig. 22 |
| B2:KLH=10:1 | + | + | - | - | - | - | |
| B2:KLH=20:1 | - | - | - | - | - | - | |
| B2:KLH=40:1 | + | - | - | - | - | - | |
| B3:KLH=5:1 | + | + | + | - | - | - | Fig. 23 |
| B3:KLH=10:1 | + | + | + | - | - | - | |
| B3:KLH=20:1 | + | + | + | - | - | - | |
| B3:KLH=40:1 | + | + | - | - | - | - | |
| B4:KLH=5:1 | + | + | + | - | - | - | Fig. 24 |
| B4:KLH=10:1 | + | + | + | - | - | - | |
| B4:KLH=20:1 | + | + | + | - | - | - | |
| B4:KLH=40:1 | + | + | - | - | - | - | |

. 2) First, the standard antibody A was diluted with normal saline by 10 times, 100 times, 1000 times, 10000 times and 100000 times, respectively, and then the diluted antibodies (with normal saline as negative control) were mixed with an equal volume of an antigen B (0.01 mg/mL) with different structures and different KLH conjugation ratios and incubated at room temperature for about 15 min. Then 40 µL of the incubated mixture was added into the above reverse ABO grouping detection card as an object to be detected. Finally, 10 µL of the indicator cells B were added, and the results were read after centrifugation. The results were shown in the following table:

.

**Table 5d**

| | Dilution times of the standard antibody A (Ab A) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| B1:KLH=5:1 | - | - | - | - | - | - | Fig. 21 |
| B1:KLH=10:1 | - | - | - | - | - | - | |
| B1:KLH=20:1 | - | - | - | - | - | - | |
| B1:KLH=40:1 | - | - | - | - | - | - | |
| B2:KLH=5:1 | - | - | - | - | - | - | Fig. 22 |
| B2:KLH=10:1 | - | - | - | - | - | - | |
| B2:KLH=20:1 | - | - | - | - | - | - | |
| B2:KLH=40:1 | - | - | - | - | - | - | |
| B3:KLH=5:1 | - | - | - | - | - | - | Fig. 23 |
| B3:KLH=10:1 | - | - | - | - | - | - | |
| B3:KLH=20:1 | - | - | - | - | - | - | |
| B3:KLH=40:1 | - | - | - | - | - | - | |
| B4:KLH=5:1 | - | - | - | - | - | - | Fig. 24 |
| B4:KLH=10:1 | - | - | - | - | - | - | |
| B4:KLH=20:1 | - | - | - | - | - | - | |
| B4:KLH=40:1 | - | - | - | - | - | - | |

. Experimental results: Table 5c showed that B2-KLH had relatively better neutralization ability for the antibody B when the antigen B (B1-B4) was conjugated to the KLH, especially the neutralization ability was the strongest when B2:KLH=20:1 (even in a high concentration environment where the antibody B was only diluted by 10 times, the antibody B could still be fully neutralized when B2:KLH=20:1). B2-KLH also showed good specificity when B2:KLH=40:1, and in a relatively high concentration environment where the antibody B was diluted by 100 times, the antibody B could still be fully neutralized. B1-KLH, B3-KLH and B4-KLH showed better neutralization ability for the antibody B when the conjugation ratio was 40:1 when compared with the same type of antigens at other conjugation ratios, and in an environment where the antibody B was diluted by 1000 times, the antibody B could be fully neutralized. Table 5d was a control experiment using the antibody A, in which the antigen B (B1-B4) conjugated to the KLH should neither perform a neutralization reaction nor react with the indicator cells B, so that the experimental result were all negative, proving that the experimental system operated normally.

### . 5.3 Detecting antibodies with the A-BSA antigen

. 1) First, the standard antibody A was diluted with normal saline by 10 times, 100 times, 1000 times, 10000 times and 100000 times, respectively, and then the diluted antibodies (with normal saline as negative control) were mixed with an equal volume of an antigen A (0.01 mg/mL) with different structures and different BSA conjugation ratios and incubated at room temperature for about 15 min. Then 40 µL of the incubated mixture was added into the above reverse ABO grouping detection card as an object to be detected. Finally, 10 µL of indicator cells A were added, and the results were read after centrifugation. The results were shown in the following table:
.

**Table 6a**

| | Dilution times of the standard antibody A (Ab A) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| A1:BSA=10:1 | + | + | + | + | + | - | Fig. 13 |
| A1:BSA=20:1 | + | + | + | + | + | - | |
| A1:BSA=40:1 | + | + | + | + | - | - | |
| A1:BSA=60:1 | + | + | + | + | - | - | |
| A1:BSA=80:1 | + | + | + | + | + | - | |
| A2:BSA=10:1 | + | - | - | - | - | - | Fig. 14 |
| A2:BSA=20:1 | + | - | - | - | - | - | |
| A2:BSA=40:1 | - | - | - | - | - | - | |
| A2:BSA=60:1 | + | - | - | - | - | - | |
| A2:BSA=80:1 | + | - | - | - | - | - | |
| A3:BSA=10:1 | + | + | - | - | - | - | Fig. 15 |
| A3:BSA=20:1 | + | + | + | + | - | - | |
| A3:BSA=40:1 | + | + | - | - | - | - | |
| A3:BSA=60:1 | + | + | - | - | - | - | |
| A3:BSA=80:1 | + | + | - | - | - | - | |
| A4:BSA=10:1 | + | + | - | - | - | - | Fig. 16 |
| A4:BSA=20:1 | + | + | + | - | - | - | |
| A4:BSA=40:1 | + | + | - | - | - | - | |
| A4:BSA=60:1 | + | + | - | - | - | - | |
| A4:BSA=80:1 | + | + | - | - | - | - | |

. 2) First, the standard antibody B was diluted with normal saline by 10 times, 100 times, 1000 times, 10000 times and 100000 times, respectively, and then the diluted antibodies (with normal saline as negative control) were mixed with an equal volume of the antigen A (0.01 mg/mL) with different structures and different BSA conjugation ratios and incubated at room temperature for about 15 min. Then 40 µL of the incubated mixture was added into the above reverse ABO grouping detection card as an object to be detected. Finally, 10 µL of indicator cells A were added, and the results were read after centrifugation. The results were shown in the following table:

.

**Table 6b**

| | Dilution times of the standard antibody B (Ab B) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| A1:BSA=10:1 | - | - | - | - | - | - | Fig. 13 |
| A1:BSA=20:1 | - | - | - | - | - | - | |
| A1:BSA=40:1 | - | - | - | - | - | - | |
| A1:BSA=60:1 | - | - | - | - | - | - | |
| A1:BSA=80:1 | - | - | - | - | - | - | |
| A2:BSA=10:1 | - | - | - | - | - | - | Fig. 14 |
| A2:BSA=20:1 | - | - | - | - | - | - | |
| A2:BSA=40:1 | - | - | - | - | - | - | |
| A2:BSA=60:1 | - | - | - | - | - | - | |
| A2:BSA=80:1 | - | - | - | - | - | - | |
| A3:BSA=10:1 | - | - | - | - | - | - | Fig. 15 |
| A3:BSA=20:1 | - | - | - | - | - | - | |
| A3:BSA=40:1 | - | - | - | - | - | - | |
| A3:BSA=60:1 | - | - | - | - | - | - | |
| A3:BSA=80:1 | - | - | - | - | - | - | |
| A4:BSA=10:1 | - | - | - | - | - | - | Fig. 16 |
| A4:BSA=20:1 | - | - | - | - | - | - | |
| A4:BSA=40:1 | - | - | - | - | - | - | |
| A4:BSA=60:1 | - | - | - | - | - | - | |
| A4:BSA=80:1 | - | - | - | - | - | - | |

### . Experimental results:

. Table 6a showed that A2-BSA had better neutralization ability for the antibody A when the antigen A (A1-A4) was conjugated to the BSA, especially the neutralization ability was the strongest when A2:BSA=40:1 (even in a high concentration environment where the antibody A was only diluted by 10 times, the antibody A could still be fully neutralized when A2:BSA=40:1). A2-BSA also showed good specificity at other conjugation ratios (10:1-20:1, 60:1-80:1), and in a relatively high concentration environment where the antibody A was diluted by 100 times, the antibody A could still be fully neutralized. A3-BSA and A4-BSA showed better neutralization ability for the antibody A when the conjugation ratio was in a range of 40:1-80:1 when compared with the same type of antigens at other conjugation ratios, and in an environment where the antibody A was diluted by 1000 times, the antibody A could be fully neutralized. Table 6b was a control experiment using the antibody B, in which the antigen A (A1-A4) conjugated to the KLH should neither perform a neutralization reaction nor react with the indicator cells A, so that the experimental results were all negative, proving that the experimental system operated normally.

### . 5.4 Detecting antibodies with the A-KLH antigen

. 1) First, the standard antibody A was diluted with normal saline by 10 times, 100 times, 1000 times, 10000 times and 100000 times, respectively, and then the diluted antibodies (with normal saline as negative control) were mixed with an equal volume of an antigen A (0.01 mg/mL) with different structures and different KLH conjugation ratios and incubated at room temperature for about 15 min. Then 40 µL of the incubated mixture was added into the above reverse ABO grouping detection card as an object to be detected. Finally, 10 µL of indicator cells A were added, and the results were read after centrifugation. The results were shown in the following table:
.

**Table 6c**

| | Dilution times of the standard antibody A (Ab A) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| A1:KLH=10:1 | + | + | + | + | + | - | Fig. 9 |
| A1:KLH=20:1 | + | + | + | + | - | - | |
| A1:KLH=40:1 | + | + | + | + | - | - | |
| A1:KLH=60:1 | + | + | + | + | - | - | |
| A1:KLH=80:1 | + | + | + | + | - | - | |
| A2:KLH=10:1 | + | - | - | - | - | - | Fig. 10 |
| A2:KLH=20:1 | + | - | - | - | - | - | |
| A2:KLH=40:1 | - | - | - | - | - | - | |
| A2:KLH=60:1 | + | - | - | - | - | - | |
| A2:KLH=80:1 | + | - | - | - | - | - | |
| A3:KLH=10:1 | + | + | - | - | - | - | Fig. 11 |
| A3:KLH=20:1 | + | + | - | - | - | - | |
| A3:KLH=40:1 | + | + | - | - | - | - | |
| A3:KLH=60:1 | + | + | - | - | - | - | |
| A3:KLH=80:1 | + | + | - | - | - | - | |
| A4:KLH=10:1 | + | + | - | - | - | - | Fig. 12 |
| A4:KLH=20:1 | + | + | - | - | - | - | |
| A4:KLH=40:1 | + | + | - | - | - | - | |
| A4:KLH=60:1 | + | + | - | - | - | - | |
| A4:KLH=80:1 | + | + | - | - | - | - | |

. 2) First, the standard antibody B was diluted with normal saline by 10 times, 100 times, 1000 times, 10000 times and 100000 times, respectively, and then the diluted antibodies (with normal saline as negative control) were mixed with an equal volume of the antigen A (0.01 mg/mL) with different structures and different KLH conjugation ratios and incubated at room temperature for about 15 min. Then 40 µL of the incubated mixture was added into the above reverse ABO grouping detection card as an object to be detected. Finally, 10 µL of the indicator cells A were added, and the results were read after centrifugation. The results were shown in the following table:

.

**Table 6d**

| | Dilution times of the standard antibody B (Ab B) with normal saline | | | | | Negative control | |
|---|---|---|---|---|---|---|---|
| Conjugation ratios | 10 | 100 | 1000 (1K) | 10000 (1W) | 100000 (10W) | Normal saline (NS) | Drawings |
| A1:KLH=10:1 | - | - | - | - | - | - | Fig. 9 |
| A1:KLH=20:1 | - | - | - | - | - | - | |
| A1:KLH=40:1 | - | - | - | - | - | - | |
| Al:KLH=60:1 | - | - | - | - | - | - | |
| A1:KLH=80:1 | - | - | - | - | - | - | |
| A2:KLH=10:1 | - | - | - | - | - | - | Fig. 10 |
| A2:KLH=20:1 | - | - | - | - | - | - | |
| A2:KLH=40:1 | - | - | - | - | - | - | |
| A2:KLH=60:1 | - | - | - | - | - | - | |
| A2:KLH=80:1 | - | - | - | - | - | - | |
| A3:KLH=10:1 | - | - | - | - | - | - | Fig. 11 |
| A3:KLH=20:1 | - | - | - | - | - | - | |
| A3:KLH=40:1 | - | - | - | - | - | - | |
| A3:KLH=60:1 | - | - | - | - | - | - | |
| A3:KLH=80:1 | - | - | - | - | - | - | |
| A4:KLH=10:1 | - | - | - | - | - | - | Fig. 12 |
| A4:KLH=20:1 | - | - | - | - | - | - | |
| A4:KLH=40:1 | - | - | - | - | - | - | |
| A4:KLH=60:1 | - | - | - | - | - | - | |
| A4:KLH=80:1 | - | - | - | - | - | - | |

. Experimental results: Table 6c showed that A2-KLH had better neutralization ability for the antibody A when the antigen A (A1-A4) was conjugated to the KLH, especially the neutralization ability was the strongest when A2:KLH=40:1 (even in a high concentration environment where the antibody A was only diluted by 10 times, the antibody A could still be fully neutralized when A2:KLH=40:1). A2-KLH also showed good specificity at other conjugation ratios (10:1-20:1, 60:1-80:1), and in a relatively high concentration environment where the antibody A was diluted by 100 times, the antibody A could still be fully neutralized. A3-KLH and A4-KLH also showed certain neutralization ability for the antibody A at various conjugation ratios (10:1-80:1), and in an environment where the antibody A was diluted by 1000 times, the antibody A could be fully neutralized. Table 6d was a control experiment using the antibody B, in which the antigen A (A1-A4) conjugated to the KLH should neither perform a neutralization reaction nor react with the indicator cells A, so that the experimental results were all negative, proving that the experimental system operated normally.

. In the above experiments, the blood group antigenic trisaccharide B analogue-protein conjugates with obvious or relatively obvious experimental effects were summarized in Table 7a. The blood group antigenic trisaccharide A analogue-protein conjugates with obvious or relatively obvious experimental effects were summarized in Table 7b.

.

**Table 7a**

| Experiment types | Blood group antigenic trisaccharide analogue-protein conjugates | | Conjugation ratios with obvious experimental effects |
|---|---|---|---|
| Antigen immobilization experiment (immunochromatography) | B-BSA | B1-BSA | None |
| | | B2-BSA | 20:1 |
| | | B3-BSA | None |
| | | B4-BSA | None |
| | B-KLH | B1-KLH | 40:1 |
| | | B2-KLH | 5:1 - 40:1 |
| | | B3-KLH | 40:1 |
| | | B4-KLH | 40:1 |
| Optical density | B-BSA | B1-BSA | None |
| determination | | B2-BSA | 20:1 |
| | | B3-BSA | 20:1 |
| | | B4-BSA | None |
| | B-KLH | B1-KLH | 40:1 |
| | | B2-KLH | 5:1 - 40:1 |
| | | B3-KLH | 40:1 |
| | | B4-KLH | 40:1 |
| Column agglutination and neutralization experiment | B-BSA | B1-BSA | None |
| | | B2-BSA | 20:1 |
| | | B3-BSA | None |
| | | B4-BSA | None |
| | B-KLH | B1-KLH | 40:1 |
| | | B2-KLH | 10:1 - 40:1 |
| | | B3-KLH | 40:1 |
| | | B4-KLH | 40:1 |

.

**Table 7b**

| Experiment types | Blood group antigenic trisaccharide analogue-protein conjugates | | Conjugation ratios with obvious experimental effects |
|---|---|---|---|
| Antigen immobilization experiment (immunochromatography) | A-BSA | A1-BSA | None |
| | | A2-BSA | 10:1 - 80:1 |
| | | A3-BSA | 10:1, 40:1 - 80:1 |
| | | A4-BSA | 10:1, 40:1 - 80:1 |
| | A-KLH | A1-KLH | None |
| | | A2-KLH | 10:1 - 80:1 |
| | | A3-KLH | 10:1, 40:1 - 80:1 |
| | | A4-KLH | 10:1, 40:1 - 80:1 |
| Optical density determination | A-BSA | A1-BSA | None |
| | | A2-BSA | 10:1 - 80:1 |
| | | A3-BSA | 10:1, 40:1 - 80:1 |
| | | A4-BSA | 10:1, 40:1 - 80:1 |
| | A-KLH | A1-KLH | None |
| | | A2-KLH | 10:1 - 80:1 |
| | | A3-KLH | 10:1 - 80:1 |
| | | A4-KLH | 10:1 - 80:1 |
| Column agglutination and neutralization experiment | A-BSA | A1-BSA | None |
| | | A2-BSA | 10:1 - 80:1 |
| | | A3-BSA | 10:1, 40:1 - 80:1 |
| | | A4-BSA | 10:1, 40:1 - 80:1 |
| | A-KLH | A1-KLH | None |
| | | A2-KLH | 10:1 - 80:1 |
| | | A3-KLH | 10:1 - 80:1 |
| | | A4-KLH | 10:1 - 80:1 |

. The embodiments of the present invention are described above with reference to the accompanying drawings, but the present invention is not limited to the aforementioned specific embodiments. The aforementioned embodiments are merely illustrative and not limiting. For those of ordinary skill in the art, many forms can be made under the teaching of present invention without departing from the scope of the claims.

## Claims

1. *In vitro* use of a blood group antigenic trisaccharide conjugate as a reagent for the detection of blood group antibodies, wherein the blood group antigenic trisaccharide conjugate is a blood group antigenic trisaccharide analogue-protein conjugate; the blood group antigenic trisaccharide analogue includes a blood group antigenic trisaccharide A analogue; the blood group antigenic trisaccharide A analogue is conjugated to the keyhole limpet hemocyanin or the bovine serum albumin to form a blood group antigenic trisaccharide A analogue-protein conjugate; and the blood group antigenic trisaccharide A analogue is selected from a group consisting of a type A2, a type A3 and a type A4, with following chemical formulas: 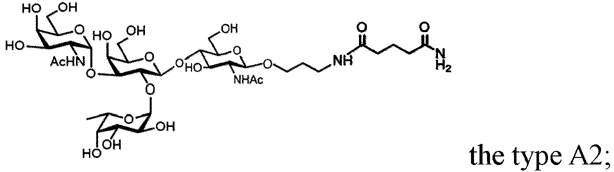 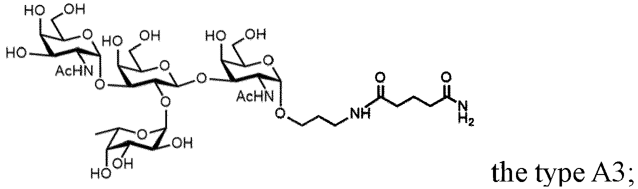 and 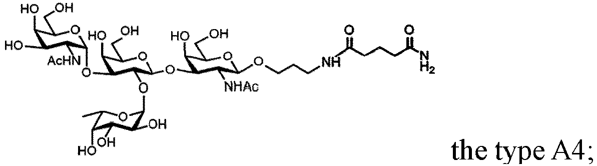 wherein, when the blood group antigenic trisaccharide A analogue is the type A2, a conjugation ratio of the blood group antigenic trisaccharide A analogue to the keyhole limpet hemocyanin is 10:1 - 80:1; and when the blood group antigenic trisaccharide A analogue is the type A3 or the type A4, a conjugation ratio of the blood group antigenic trisaccharide A analogue to the keyhole limpet hemocyanin is 10:1 - 80:1;wherein, when the blood group antigenic trisaccharide A analogue is the type A2, a conjugation ratio of the blood group antigenic trisaccharide A analogue to the bovine serum albumin is 10:1 - 80:1; and when the blood group antigenic trisaccharide A analogue is the type A3 or the type A4, a conjugation ratio of the blood group antigenic trisaccharide A analogue to the bovine serum albumin is 10:1 or 40:1-80:1.

2. The use according to claim 1, wherein, the blood group antibody detection reagent is used for immunochromatographic detection, optical density detection and column agglutination detection.

3. The use according to claim 2, wherein, the blood group antibody detection reagent is used for the immunochromatographic detection; and when the blood group antigenic trisaccharide A analogue is the type A3 or the type A4, a conjugation ratio of the blood group antigenic trisaccharide A analogue to the keyhole limpet hemocyanin is 10:1 or 40:1-80:1.

4. An immunochromatographic detection kit, comprising a detection card, wherein, the detection card uses a nitrocellulose membrane; the detection card is coated with a conjugate of a blood group antigenic trisaccharide A analogue to the keyhole limpet hemocyanin or the bovine serum albumin, suitable for use in detecting a blood group antibody in serum sample; and the blood group antigenic trisaccharide A analogue is selected from a group consisting of a type A2, a type A3 and a type A4, with following chemical formulas: 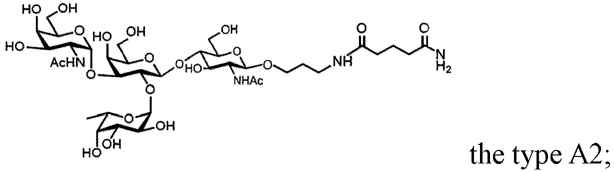 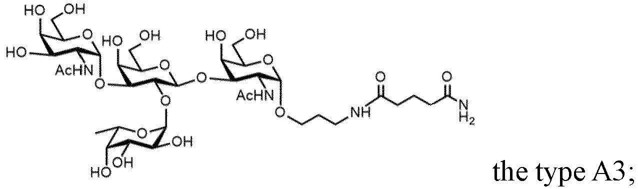 and 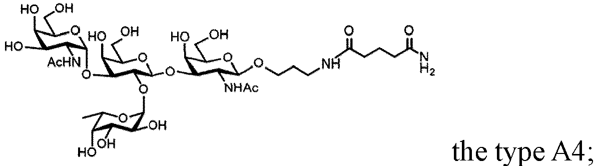
wherein, when the blood group antigenic trisaccharide A analogue is the type A2, a conjugation ratio of the blood group antigenic trisaccharide A analogue to the keyhole limpet hemocyanin is 10:1 - 80:1; and when the blood group antigenic trisaccharide A analogue is the type A3 or the type A4, a conjugation ratio of the blood group antigenic trisaccharide A analogue to the keyhole limpet hemocyanin is 10:1 or 40:1-80:1;
wherein, when the blood group antigenic trisaccharide A analogue is the type A2, a conjugation ratio of the blood group antigenic trisaccharide A analogue to the bovine serum albumin is 10:1 - 80:1; and when the blood group antigenic trisaccharide A analogue is the type A3 or the type A4, a conjugation ratio of the blood group antigenic trisaccharide A analogue to the bovine serum albumin is 10:1 or 40:1-80:1.

5. A column agglutination kit, wherein, comprising a reverse ABO grouping detection card and a blood group antigenic trisaccharide analogue-protein conjugate, wherein, the blood group antigenic trisaccharide analogue-protein conjugate includes a conjugate of a blood group antigenic trisaccharide A analogue to the keyhole limpet hemocyanin or the bovine serum albumin; the blood group antigenic trisaccharide A analogue is selected from a group consisting of a type A2, a type A3 and a type A4; when the blood group antigenic trisaccharide A analogue is the type A2, a conjugation ratio of the blood group antigenic trisaccharide A analogue to the keyhole limpet hemocyanin or the bovine serum albumin is 10:1 - 80:1; when the blood group antigenic trisaccharide A analogue is the type A3 or the type A4, a conjugation ratio of the blood group antigenic trisaccharide A analogue to the keyhole limpet hemocyanin is 10:1 - 80:1, and a conjugation ratio of the blood group antigenic trisaccharide A analogue to the bovine serum albumin is 10:1 or 40:1-80:1; and chemical formulas of the type A2, the type A3 and the type A4 are as follows: 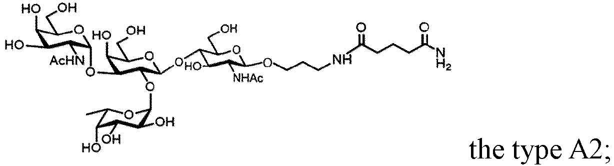 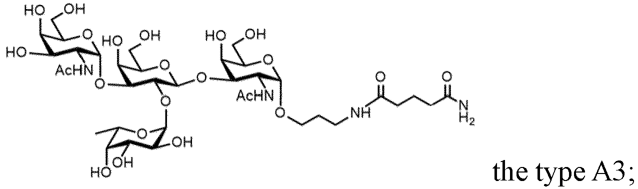 and

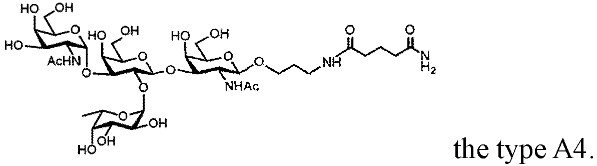

## Patentansprüche

1. *In-vitro-Verwendung* eines Blutgruppen-antigenen Trisaccharid-Konjugats als ein Reagenz für den Nachweis von Blutgruppen-Antikörpern, wobei das Blutgruppen-antigene Trisaccharid-Konjugat ein Blutgruppen-antigenes Trisaccharid-Analogon-Protein-Konjugat ist; das Blutgruppen-antigene Trisaccharid-Analogon ein Blutgruppen-antigenes Trisaccharid-A-Analogon einschließt; das Blutgruppen-antigene Trisaccharid-A-Analogon mit dem Schlüssellochschnecken-Hämocyanin oder dem Rinderserumalbumin konjugiert wird, um ein Blutgruppen-antigenes Trisaccharid-A-Analogon-Protein-Konjugat auszubilden; und das Blutgruppen-antigene Trisaccharid-A-Analogon aus einer Gruppe ausgewählt ist, bestehend aus einem Typ A2, einem Typ A3 und einem Typ A4, mit den folgenden chemischen Formeln: 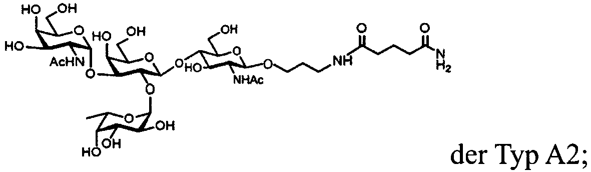 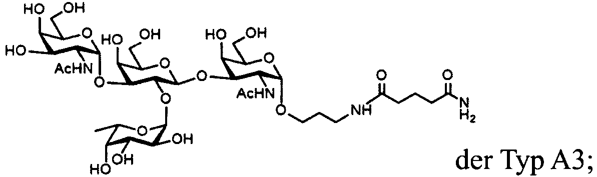 und 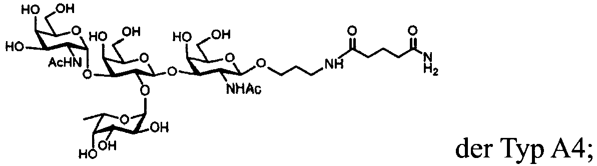 wobei, wenn das Blutgruppen-antigene Trisaccharid-A-Analogon der Typ A2 ist, das Konjugationsverhältnis des Blutgruppen-antigenen Trisaccharid-A-Analogons zu dem Schlüssellochschnecken-Hämocyanin 10 : 1-80 : 1 beträgt; und wenn das Blutgruppen-antigene Trisaccharid-A-Analogon der Typ A3 oder der Typ A4 ist, das Konjugationsverhältnis des Blutgruppen-antigenen Trisaccharid-A-Analogons zu dem Schlüssellochschnecken-Hämocyanin 10 : 1-80 : 1 beträgt, wobei, wenn das Blutgruppen-antigene Trisaccharid-A-Analogon der Typ A2 ist, ein Konjugationsverhältnis des Blutgruppen-antigenen Trisaccharid-A-Analogons zu dem Rinderserumalbumin 10 : 1-80 : 1 beträgt; und wenn das Blutgruppen-antigene Trisaccharid-A-Analogon der Typ A3 oder der Typ A4 ist, ein Konjugationsverhältnis des Blutgruppen-antigenen Trisaccharid-A-Analogons zu dem Rinderserumalbumin 10 : 1 oder 40 : 1-80 : 1 beträgt;

2. Verwendung nach Anspruch 1, wobei das Blutgruppen-Antikörper-Nachweisreagenz für einen immunchromatographischen Nachweis, einen Nachweis der optischen Dichte und eine Säulenagglutinationsnachweis verwendet wird.

3. Verwendung nach Anspruch 2, wobei das Blutgruppen-Antikörper-Nachweisreagenz für den immunochromatographische Nachweis verwendet wird; und wenn das Blutgruppen-antigene Trisaccharid-A-Analogon der Typ A3 oder der Typ A4 ist, ein Konjugationsverhältnis des Blutgruppen-antigenen Trisaccharid-A-Analogons zu dem Schlüssellochschnecken-Hämocyanin 10 : 1 oder 40 : 1-80 : 1 beträgt;

4. Kit für immunochromatographischen Nachweis, umfassend eine Nachweiskarte, wobei die Nachweiskarte eine Nitrocellulosemembran verwendet; die Nachweiskarte mit einem Konjugat eines Blutgruppen-antigenen Trisaccharid-A-Analogons mit dem Schlüssellochschnecken-Hämocyanin oder dem Rinderserumalbumin beschichtet ist, das für die Verwendung bei dem Nachweisen eines Blutgruppen-Antikörpers in einer Serumprobe geeignet ist; und das Blutgruppen-antigene Trisaccharid-A-Analogon aus einer Gruppe ausgewählt ist, bestehend aus einem Typ A2, einem Typ A3 und einem Typ A4, mit den folgenden chemischen Formeln: 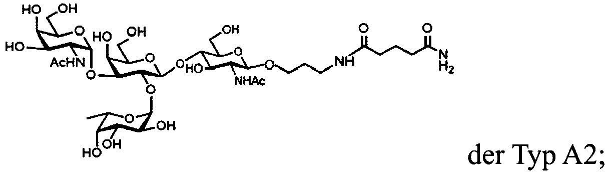 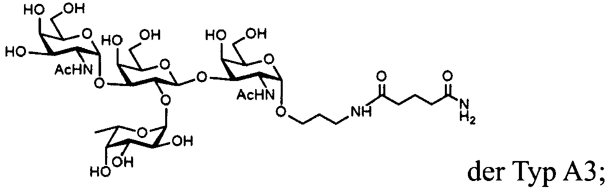 und 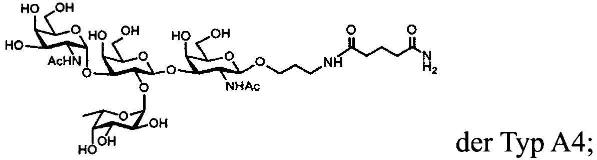
wobei, wenn das Blutgruppen-antigene Trisaccharid-A-Analogon der Typ A2 ist, das Konjugationsverhältnis des Blutgruppen-antigenen Trisaccharid-A-Analogons zu dem Schlüssellochschnecken-Hämocyanin 10 : 1-80 : 1 beträgt; und wenn das Blutgruppen-antigene Trisaccharid-A-Analogon der Typ A3 oder der Typ A4 ist, ein Konjugationsverhältnis des Blutgruppen-antigenen Trisaccharid-A-Analogons zu dem Schlüssellochschnecken-Hämocyanin 10 : 1 oder 40 : 1-80 : 1 beträgt;
wobei, wenn das Blutgruppen-antigene Trisaccharid-A-Analogon der Typ A2 ist, ein Konjugationsverhältnis des Blutgruppen-antigenen Trisaccharid-A-Analogons zu dem Rinderserumalbumin 10 : 1-80 : 1 beträgt; wenn das Blutgruppen-antigene Trisaccharid-A-Analogon der der Typ A3 oder der Typ A4 ist, das Konjugationsverhältnis des Blutgruppen-antigenen Trisaccharid-A-Analogons zu dem Rinderserumalbumin 10 : 1 oder 40 : 1-80 : 1 beträgt;

5. Säulenagglutinationskit, wobei, umfassend eine Nachweiskarte für die umgekehrte ABO-Gruppierung und ein Blutgruppen-antigenes Trisaccharid-Analogon-Protein-Konjugat, wobei das Blutgruppen-antigene Trisaccharid-Analogon-Protein-Konjugat ein Konjugat eines Blutgruppen-antigenen Trisaccharid-A-Analogons mit dem Schlüssellochschnecken-Hämocyanin oder dem Rinderserumalbumin einschließt; das Blutgruppen-antigene Trisaccharid-A-Analogon aus einer Gruppe ausgewählt ist, bestehend aus einem einem Typ A2, einem Typ A3 und einem Typ A4; wenn das Blutgruppen-antigene Trisaccharid-A-Analogon der Typ A2 ist, ein Konjugationsverhältnis des Blutgruppen-antigenen Trisaccharid-A-Analogons zu dem Schlüssellochschnecken-Hämocyanin oder dem Rinderserumalbumin 10 : 1-80 : 1 beträgt; wenn das Blutgruppen-antigene Trisaccharid-A-Analogon der Typ A3 oder der A4 ist, ein Konjugationsverhältnis des Blutgruppen-antigenen Trisaccharid-A-Analogons zu dem Schlüssellochschnecken-Hämocyanin 10 : 1-80 : 1 beträgt und ein Konjugationsverhältnis des Blutgruppen-antigenen Trisaccharid-A-Analogons zu dem Rinderserumalbumin 10 : 1 oder 40 : 1-80 : 1 beträgt; und die chemischen Formeln des Typs A2, des Typs A3 und des Typs A4 wie folgt lauten: 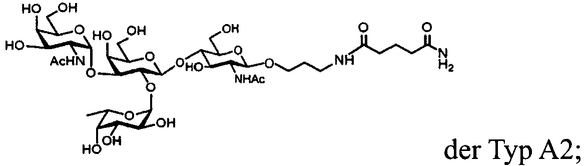 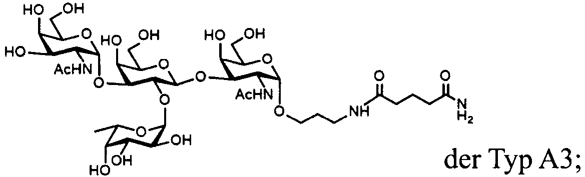 und 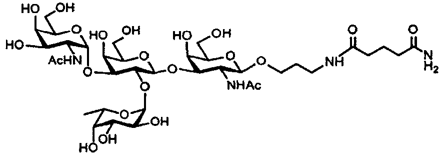 der Typ A4;

## Revendications

1. *Utilisation in vitro*
d'un conjugué de trisaccharide antigénique de groupe sanguin en tant que réactif pour la détection d'anticorps de groupe sanguin, dans laquelle le conjugué trisaccharide antigénique de groupe sanguin est un conjugué analogue de trisaccharide antigénique de groupe sanguin-protéine ; l'analogue de trisaccharide antigénique de groupe sanguin comporte un analogue A de trisaccharide antigénique de groupe sanguin ; l'analogue A de trisaccharide antigénique de groupe sanguin est conjugué à l'hémocyanine de patelle ou à l'albumine de sérum bovin pour former un conjugué analogue A de trisaccharide antigénique de groupe sanguin-protéine ; et l'analogue A de trisaccharide antigénique de groupe sanguin est choisi dans un groupe constitué d'un type A2, d'un type A3 et d'un type A4, avec les formules chimiques suivantes : 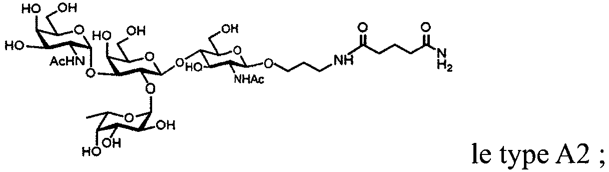 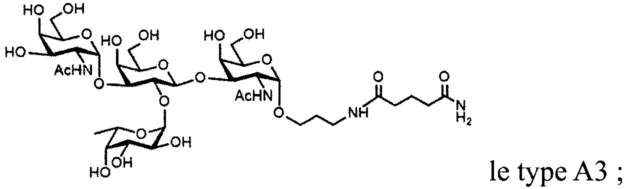 et 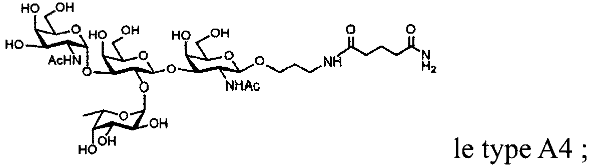
dans laquelle, lorsque l'analogue A de trisaccharide antigénique de groupe sanguin est le type A2, un rapport de conjugaison entre l'analogue A de trisaccharide antigénique de groupe sanguin et l'hémocyanine de patelle est de 10:1 à 80:1 ; et lorsque l'analogue A de trisaccharide antigénique de groupe sanguin est le type A3 ou le type A4, un rapport de conjugaison entre l'analogue A de trisaccharide antigénique de groupe sanguin et l'hémocyanine de patelle est de 10:1 à 80:1 ;dans laquelle, lorsque l'analogue A de trisaccharide antigénique de groupe sanguin est le type A2, un rapport de conjugaison entre l'analogue A de trisaccharide antigénique de groupe sanguin et l'albumine de sérum bovin est de 10:1 à 80:1 ; et lorsque l'analogue A de trisaccharide antigénique de groupe sanguin est le type A3 ou le type A4, un rapport de conjugaison entre l'analogue A de trisaccharide antigénique de groupe sanguin et l'albumine de sérum bovin est de 10:1 ou 40:1 à 80:1.

2. Utilisation selon la revendication 1, dans laquelle le réactif de détection d'anticorps de groupe sanguin est utilisé pour une détection immunochromatographique, une détection par densité optique et une détection par agglutination sur colonne.

3. Utilisation selon la revendication 2, dans laquelle le réactif de détection d'anticorps de groupe sanguin est utilisé pour la détection immunochromatographique ; et lorsque l'analogue A de trisaccharide antigénique de groupe sanguin est le type A3 ou le type A4, un rapport de conjugaison entre l'analogue A de trisaccharide antigénique de groupe sanguin et l'hémocyanine de patelle est de 10:1 ou 40:1 à 80:1.

4. Trousse de détection immunochromatographique, comprenant une carte de détection, dans laquelle la carte de détection utilise une membrane de nitrocellulose ; la carte de détection est revêtue d'un conjugué d'un analogue A de trisaccharide antigénique de groupe sanguin à l'hémocyanine de patelle ou à l'albumine de sérum bovin, convenant pour une utilisation dans la détection d'un anticorps de groupe sanguin dans un échantillon de sérum ; et l'analogue A de trisaccharide antigénique de groupe sanguin est choisi dans un groupe constitué d'un type A2, d'un type A3 et d'un type A4, avec les formules chimiques suivantes : 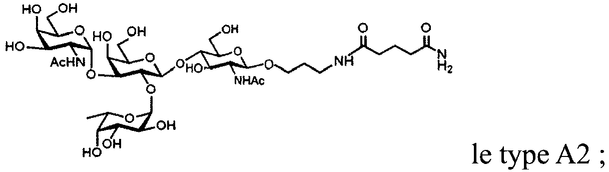 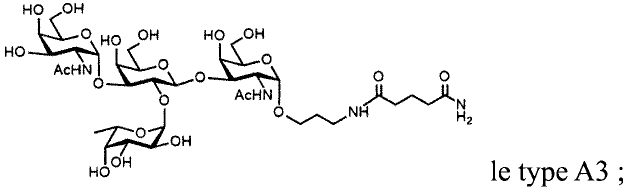 et 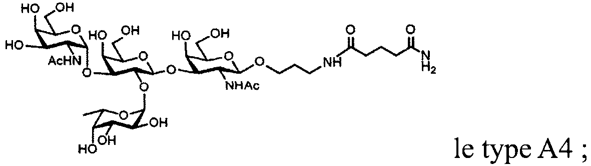
dans laquelle, lorsque l'analogue A de trisaccharide antigénique de groupe sanguin est le type A2, un rapport de conjugaison entre l'analogue A de trisaccharide antigénique de groupe sanguin et l'hémocyanine de patelle est de 10:1 à 80:1 ; et lorsque l'analogue A de trisaccharide antigénique de groupe sanguin est le type A3 ou le type A4, un rapport de conjugaison entre l'analogue A de trisaccharide antigénique de groupe sanguin et l'hémocyanine de patelle est de 10:1 ou 40:1 à 80:1 ;
dans laquelle, lorsque l'analogue A de trisaccharide antigénique de groupe sanguin est le type A2, un rapport de conjugaison entre l'analogue A de trisaccharide antigénique de groupe sanguin et l'albumine de sérum bovin est de 10:1 à 80:1 ; et lorsque l'analogue A de trisaccharide antigénique de groupe sanguin est le type A3 ou le type A4, un rapport de conjugaison entre l'analogue A de trisaccharide antigénique de groupe sanguin et l'albumine de sérum bovin est de 10:1 ou 40:1 à 80:1.

5. Trousse d'agglutination sur colonne, dans laquelle, comprenant une carte de détection de groupage ABO inverse et un conjugué analogue de trisaccharide antigénique de groupe sanguin-protéine, dans laquelle le conjugué analogue de trisaccharide antigénique de groupe sanguin-protéine comporte un conjugué d'un analogue A de trisaccharide antigénique de groupe sanguin à l'hémocyanine de patelle ou à l'albumine de sérum bovin ; l'analogue A de trisaccharide antigénique de groupe sanguin est choisi dans un groupe constitué d'un type A2, d'un type A3 et d'un type A4 ; lorsque l'analogue A de trisaccharide antigénique de groupe sanguin est le type A2, un rapport de conjugaison entre l'analogue A de trisaccharide antigénique de groupe sanguin et l'hémocyanine de patelle ou l'albumine de sérum bovin est de 10:1 à 80:1 ; lorsque l'analogue A de trisaccharide antigénique de groupe sanguin est le type A3 ou le type A4, un rapport de conjugaison entre l'analogue A de trisaccharide antigénique de groupe sanguin et l'hémocyanine de patelle est de 10:1 à 80:1, et un rapport de conjugaison entre l'analogue A de trisaccharide antigénique de groupe sanguin et l'albumine de sérum bovin est de 10:1 ou 40:1 à 80:1 ; et les formules chimiques du type A2, du type A3 et du type A4 sont les suivantes : 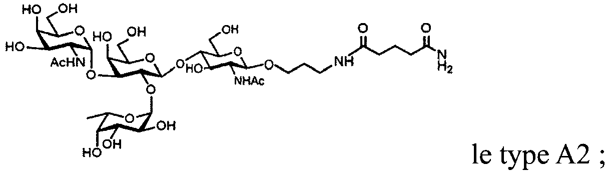 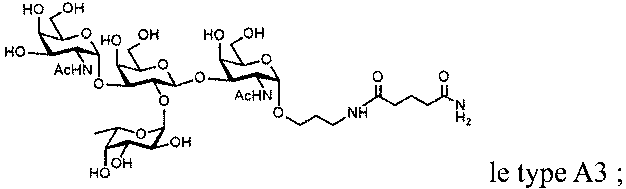 et
